# EUROPEAN PATENT SPECIFICATION

(11) **EP 4 456 826 B1**
(45) Date of publication and mention of the grant of the patent: **22.04.2026**
(21) Application number: 22802364.4
(22) Date of filing: 13.10.2022
(51) Int. Cl.: A61C 7/00, A61C 1/08, A61C 9/00

(54) **METHOD TO SEMI-AUTOMATICALLY DETERMINE VIRTUAL DENTAL OCCLUSION**
VERFAHREN ZUR HALBAUTOMATISCHEN BESTIMMUNG EINER VIRTUELLEN ZAHNOKKLUSION
MÉTHODE DE DÉTERMINATION SEMI-AUTOMATIQUE D'UNE OCCLUSION DENTAIRE VIRTUELLE

(30) Priority: 28.12.2021 US 202163266079 P
(43) Date of publication of application: 06.11.2024
(73) Proprietor: Materialise NV, 3001 Leuven (BE)
(72) Inventor: BARTELS, Ward, 3001 Leuven (BE); VAN DIJCK, Christophe, 3001 Leuven (BE)
(74) Representative: Abel & Imray LLP
(86) International application number: PCT/US2022/046533
(87) International publication number: WO 2022/266559

(56) References cited:
- WO-A1-2021/072378
- US-A1- 2002 064 746
- F. BAAN ET AL: "Virtual setup in orthodontics: planning and evaluation", CLINICAL ORAL INVESTIGATIONS, 12 November 2019 (2019-11-12), DE, XP055706708, ISSN: 1432-6981, DOI: 10.1007/s00784-019-03097-3

## Description

### BACKGROUND

### Field of the invention

Aspects of the present disclosure relate to determining occlusion. In some aspects, the present disclosure relates specifically to devices, systems and methods for determining virtual occlusion for orthognathic surgery planning.

### Description of the Related Technology

In orthognathic surgery, a surgeon performs an osteotomy to reposition the teeth of one or both jaws, aiming to alleviate clinical and esthetic problems caused by severe jaw misalignment that orthodontics alone cannot treat. Computer-assisted planning systems (such as Materialise's^{®} ProPlan CMF, SurgiCase^{®}, Mimics^{®}, and the like) can help the surgeon predict the functional and esthetic outcome of the procedure. Such planning systems may provide for 3D- printed dental splints for use during surgery to guide the teeth toward the planned position. The user of such a software system must specify the relative position and orientation of the teeth of both jaws (i.e., the maxilla with respect to the mandible, or vice versa), or parts thereof, when the patient brings both jaws into contact to bite. The relative position and orientation of the teeth of both jaws when the patient brings both jaws into contact to bite is referred to as the patient's occlusion.

In some orthognathics cases, the surgeon may need to perform additional osteotomies to split the maxilla and/or mandible into multiple parts. Each individual part is then repositioned in a slightly different way. This is referred to as a split mandible or a split maxilla. It may be required, e.g., if the maxilla or mandible is too small to support a proper occlusion or to obtain an acceptable functional or aesthetic outcome for the patient. Typically, the parts of one jaw are repositioned to be slightly further apart than they were before surgery. In such cases, the occlusion comprises the relative position and orientation of all jaw parts relative to the other jaw. If the planned intervention involves splitting both the maxilla and mandible, then the occlusion comprises the relative position and orientation of all jaw parts relative to one arbitrarily selected part in one of both jaws.

In some orthognathics cases, the surgeon may decide that during or after orthognathic surgery, material is to be removed from the occlusal surface of one or more teeth by grinding or burring. This may be the case if, for example, parts of one or more teeth protrude from the rest of the teeth in the same jaw and thereby interfere with an appropriate occlusion. In such cases, burring down the protruding parts of those teeth may improve the patient's post-operative occlusion, by allowing the teeth of both jaws to make contact in different points.

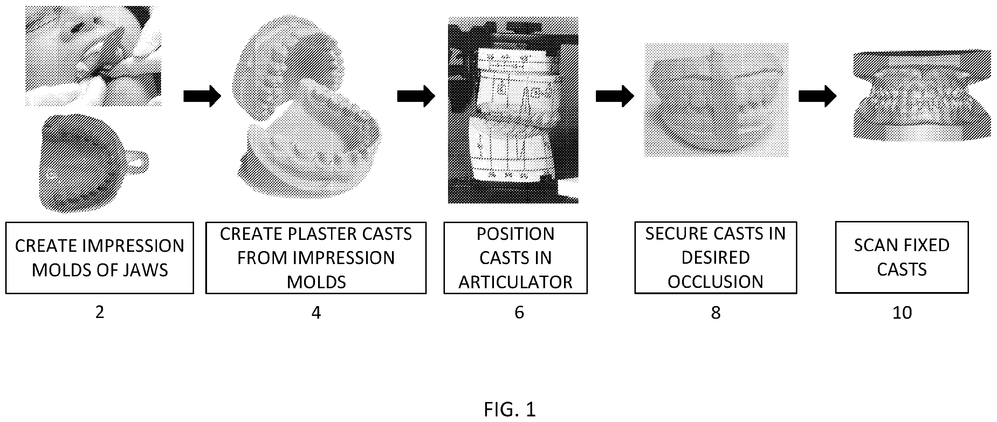

The occlusion is determined by an optical scan of dental cast models which the surgeon has physically placed in occlusion. Figure 1 depicts a conventional workflow to specify the occlusion for computer-assisted planning of orthognathic surgery. At step 2 of Figure 1, impression molds of the patient's maxilla and mandible are created. At step 4, plaster casts are created from the impression molds. At step 6, the two plaster casts are positioned together in an articulator to determine and represent the desired occlusion. At step 8, the plaster casts are secured together in the desired occlusion. At step 10, the fixed plaster casts are optically scanned for use in a computer-assisted planning system.

In split maxilla or split mandible cases, step 4 in Figure 1 additionally involves physically cutting one or both plaster casts to simulate the additional osteotomies performed to split the corresponding jaw(s). The resulting parts of each cast are then fixed together in a different relative position and orientation, representing the desired new shape of the split jaw.

However, in many cases, such "occlusion scans" may not be readily available. For example, the surgeon may prefer a fully digital workflow using intra-oral scans of the teeth instead of casts. As another example, the relative positioning of the casts (representing the desired occlusion) may be disturbed prior to optical scanning, such as during transport.

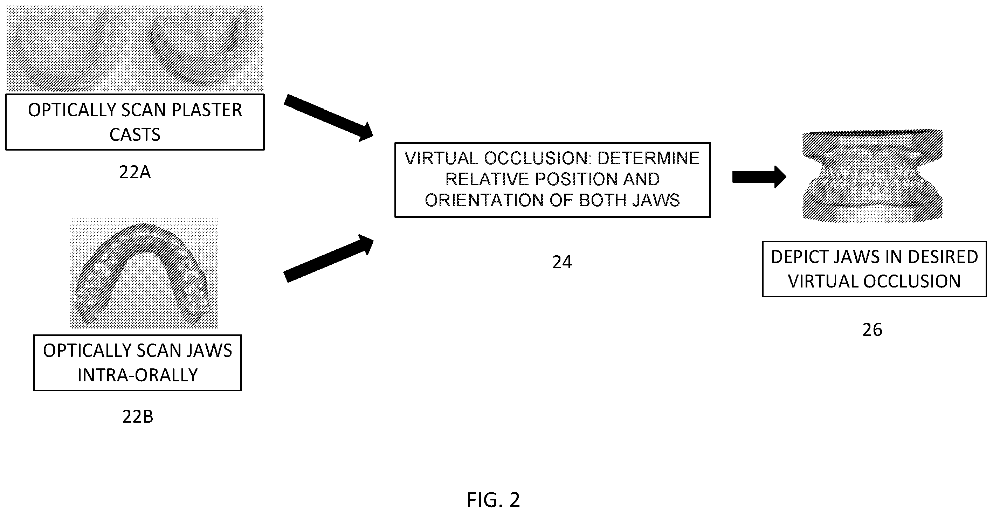

In such cases, the surgeon would need to specify the occlusion through other means, such as by using a user interface integrated into the planning software to manually rotate and translate 3-D models of the jaws and/or jaw parts relative to one another until the desired occlusion is achieved. Figure 2 depicts an example all-virtual workflow to specify the occlusion for computer-assisted planning of orthognathic surgery. At step 22A, the plaster casts (created as described above in connection with steps 2 and 4 of Figure 1) are optically scanned. Alternatively, at step 22B, both jaws are optically scanned intra-orally. At step 24, the optical scans are provided to the planning software, and a user of the planning software (e.g., the surgeon) manipulates the user interface to determine a desired occlusion. At step 26, the planning software depicts the jaws in the desired occlusion for surgical planning. The depiction of the desired occlusion with the planning software is called a virtual occlusion herein.

For split mandible or split maxilla cases, fully specifying the occlusion requires determining the relative position and orientation of all jaw parts relative to one part of one jaw. Therefore, the user of the planning software needs to manually rotate and translate 3-D models of all jaw parts except one. This may be the same process as that used to specify the occlusion of a jaw that is not split, repeated multiple times.

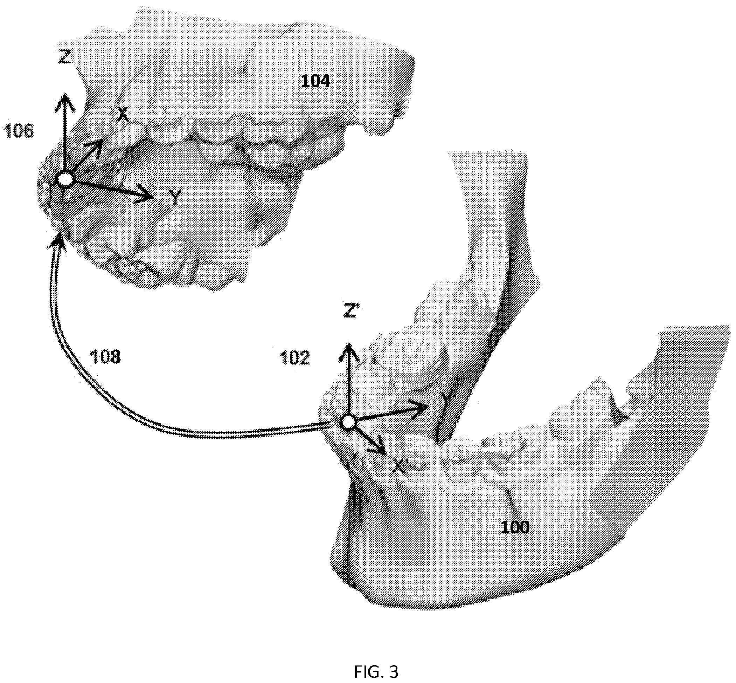

As will be understood, the occlusion is defined by the position and rotation of a moving jaw relative to a coordinate system with its origin fixed with respect to a stationary jaw. Referring to Figure 3, and as discussed herein, the coordinate system of moving jaw portion 100 is referred as the Local Coordinate System (LCS) 102 having orthogonal X', Y', and Z' axes. The coordinate system of the stationary jaw portion 104 is referred to as the World Coordinate System (WCS) 106 having orthogonal X, Y, and Z axes. The occlusion 108 can be mathematically defined by the position of the origin, and directions of the axes, of the LCS 102 attached to the moving jaw portion 100, as defined in the WCS 106.

In this manner, the position of the moving jaw portion 100 can be identified with X, Y, Z coordinates in the WCS 106. Further, the orientation of the moving jaw portion 100 can be identified with degrees of rotation about the X, Y and Z axes in the WCS 106. Thus, the occlusion 108 is the position and orientation of the moving jaw portion 100 as defined in six degrees of freedom (three positional and three rotational) with respect to the WCS 106.

The definition of coordinate systems is arbitrary. That is, as discussed herein and shown in Figure 3, the mandible may be defined as the moving jaw portion 100 to which the LCS 102 is attached, and the maxilla may be defined as the stationary jaw portion 104 to which the WCS 106 is attached. Alternatively, the mandible could instead be identified as the stationary jaw portion 104 to which the WCS 106 is attached, and the maxilla could instead be identified as the moving jaw portion 100 to which the LCS 102 is attached.

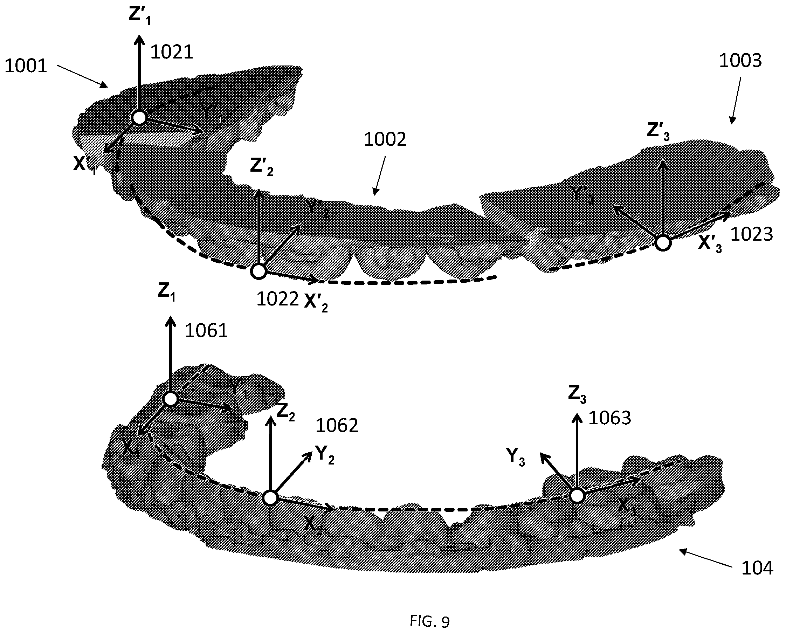

For split mandible or split maxilla cases, as is illustrated in Figure 9, the jaw 104 that is not split may be considered the stationary jaw portion to which the WCS 1061, 1062, 1063 is attached, while each part 1001, 1002, 1003 of the split jaw may be considered a separate moving jaw portion with its own LCS 1021, 1022, 1023. If only one jaw is split, the occlusion can be fully specified by defining the LCS of all parts of that jaw. If both the maxilla and mandible are split, the occlusion can be defined iteratively. For example, first the parts of the mandible can be merged into a single object which is considered the stationary jaw portion while the parts of the maxilla are each considered as separate moving jaw portions with their own LCS that can be moved and rotated individually. Subsequently, the parts of the maxilla can be merged and considered the stationary jaw portion, which has the effect of fixing the relative positions and orientations of the maxilla parts, and the parts of the mandible can be considered as separate moving jaw portions to be manipulated individually. This process can be repeated, possibly under control of the user of the planning software. Alternatively, this iterative process could start with the parts of the maxilla merged into a single stationary jaw portion while the individual parts of the mandible are moved - reversing the order in which mandible parts and maxilla parts are manipulated.

While the origin and orientation of the WCS 106 and the LCS 102 can be arbitrarily defined, it may also be convenient to define the X-Y plane of the WCS 106 as the plane tangential to the teeth surfaces of the fixed jaw portion 104. Similarly, it may be convenient to define the Z axis of the WCS 106 as orthogonal to the X-Y plane through the dental midline of the fixed jaw portion 104. Likewise, it may be convenient to define the X'-Y' plane of the LCS 102 as the plane tangential to the teeth surfaces of the moving jaw portion 100. Similarly, it may be convenient to define the Z' axis of the LCS 102 as orthogonal to the X'-Y' plane through the dental midline of the moving jaw portion 100.

For split mandible or split maxilla cases, one or more parts of the split jaw may consist of several adjacent teeth that form an almost straight row, resulting in the part having a very elongated shape. In such cases, it may be convenient to define the X'-Y' plane of the corresponding LCS as the plane tangential to the teeth surfaces of the split jaw. Furthermore, it may be convenient to align either the X' axis or the Y' axis of the LCS to be parallel with the direction of the row of teeth that the part represents; this is equivalent to aligning the X' axis or Y' axis, respectively, to be tangential to the dental arc. By consequence, the Y' axis or the X' axis, respectively, will be perpendicular to the dental arc and to the direction of the row of teeth. Similarly, it may be convenient to define the Z' axis of the LCS as orthogonal to the X'-Y' plane, passing through the middle (defined, for example, as the center of gravity) of the jaw part. It may also be convenient to define the WCS in a matching way for the opposite, stationary jaw portion, with the X axis or the Y axis tangential to the dental arc; respectively, the Y axis or the X axis perpendicular to the dental arc; the X-Y plane tangential to the teeth surfaces of the stationary jaw; and the Z axis passing through the middle of the split part represented by the moving jaw portion (in its initial position). Using such largely coinciding coordinate systems for the stationary jaw (part) and the moving jaw (part) makes it easier for a user to interpret the translation and rotation values. Figure 9 shows an example of a split maxilla case, with the teeth of the mandible 104 representing one jaw portion and the teeth of the maxilla split into three parts 1001, 1002, 1003. In this example the mandible is the fixed jaw portion and each maxilla part is a separate moving jaw portion. A separate LCS 1021, 1022, 1023 comprising an origin, indicated by a dot, and three axes X'₁, Y'₁, Z'₁; X'₂, Y'₂, Z'₂; X'₃, Y'₃, Z'₃ is defined for each maxilla part. The axes X'₁, X'₂, and X'₃ are tangential to the dental arc (dashed lines). The axes Y'₁, Y'₂, and Y'₃ are perpendicular to the dental arc and parallel to a horizontal plane tangential to the teeth surfaces. The axes Z'₁, Z'₂, and Z'₃ are perpendicular to said horizontal plane. For determining the occlusion for a particular maxilla part, a corresponding WCS is defined, attached to the mandible 104. A different WCS 1061, 1062, 1063 is shown for each maxilla part. WCS axes are labelled X₁, Y₁, and Z₁ for the right part; X₂, Y₂, and Z₂ for the middle part; and X₃, Y₃, and Z₃ for the left part. The WCS definitions are similar to those for the LCS, with the axes X₁, X₂, and X₃ tangential to the dental arc (dashed line), the axes Y₁, Y₂, and Y₃ perpendicular to the dental arc and parallel to a horizontal plane tangential to the teeth surfaces, and the axes Z₁, Z₂, and Z₃ perpendicular to said horizontal plane.

One possible orthognathic surgical planning tool, e.g., surgery planning software, could give the user full control over the position and orientation of one of the jaw portions 100, 104. That is, the user controls the position of the moving jaw portion 100 in all three spatial dimensions as well as the rotation of the moving jaw portion 100 about all three primary axes. Although this gives the user complete control, it may be cumbersome to use because of the many requirements that must be taken into account when determining the occlusion 108.

Specifically, for a clinically stable occlusion 108, the teeth of both jaw portions 100, 104 should make contact in at least three points, where contact may be defined as a small overlap (intersection) of the 3-D models representing both sets of teeth, such as with an overlap distance of approximately 0.3 mm (exact value may vary by surgeon or by case). The contact points should be spaced sufficiently apart to obtain a stable occlusion 108. This may be verified by inspecting cross-sections through the 3D models of the teeth. To cover the entire surface of all teeth, a large number of these cross-sections must be checked, which makes this a tedious process requiring extensive time and/or processing resources.

Furthermore, the user needs to account for additional clinical requirements and trade-offs. For example, the dental midlines of both jaw portions 100, 104 should be aligned for aesthetic reasons. Additionally, the anterior-posterior alignment of both jaw portions 100, 104 must be set according to the surgeon's expectations. Any change the user makes to the jaw portions' relative position and orientation in the user interface will tend to simultaneously impact many of these often-conflicting requirements. Thus, after each change, the user then needs to verify whether such requirements are satisfied within acceptable ranges.

For split mandible or split maxilla cases, a surgery planning software could give the user full control over the position and orientation of all the parts of the split jaw, using methods similar to what would be used if the jaw was not split, applied to each part of the split jaw individually. However, this will have the effect of multiplying the effort required of the user, as manipulations of position and orientation must be performed for each part.

Because of the limitations of such potential solutions, and previous solutions such as described in Pongrácz, F., & Bárdosi, Z. (2006), "Dentition planning with image-based occlusion analysis," International Journal of Computer Assisted Radiology and Surgery, 1(3), 149-156; Nadjmi, N., Mollemans, W., Daelemans, A., Van Hemelen, G., Schutyser, F., and Bergé, S. (2010), "Virtual occlusion in planning orthognathic surgical procedures," International Journal of Oral and Maxillofacial Surgery 39, 457-462; and Deng, H., Yuan, P., Wong, S., Gateno, J., Garrett, F.A., Ellis, R.K., English, J.D., Jacob, H.B., Kim, D., Barber, J.C., et al. (2020), "An automatic approach to establish clinically desired final dental occlusion for one-piece maxillary orthognathic surgery," International Journal of Computer Assisted Radiology and Surgery 15(11):1763-1773, determination of virtual occlusion 108 using such solutions may only be performed by specialized clinical engineers rather than the surgeons themselves. In addition, a great deal of training may be required. Further, determining a desired virtual occlusion 108 can be a time-consuming process even for an experienced engineer: for example, a difficult case may require 15 minutes to an hour depending on the user's experience level. This further complicates the planning process, as the user may not be able to immediately apply a change to the planned occlusion 108 when the surgeon requests it.

On the other hand, other possible planning platforms could provide a fully automated approach wherein the virtual occlusion 108 is suggested and set by an algorithm. In this case, the user has little to no freedom to fine-tune the occlusion 108. This may provide for sub-optimal outcomes that do not account for factors that potentially only a surgeon could account for, such as, for example, a surgical approach, pre- and post- operative soft tissue properties, aesthetic requirements, or outcome expectations.

International PCT patent application no. WO-A-2021/072378 describes methods and systems for establishing dental occlusion. The systems and methods can be used for 1-, 2-, or 3-piece maxillary orthognathic surgeries. An example computer-implemented method includes receiving a maxillary dental model and a mandibular dental model, identifying a plurality of dental landmarks in each of the maxillary and mandibular dental models, and extracting a plurality of points-of-interest from each of the maxillary and mandibular dental models. The dental landmarks include a plurality of maxillary dental landmarks and a plurality of mandibular dental landmarks. The method further includes aligning the maxillary and mandibular points-of-interest, and fine tuning the alignment of the maxillary and mandibular dental models to achieve maximum contact with a collision constraint.

It should be noted that the information included in the Background section herein is simply meant to provide a reference for the discussion of certain embodiments in the Detailed Description. None of the information included in this Background should be considered as an admission of prior art.

### Summary of Embodiments of the Disclosure

The invention is as defined in the appended claims. In certain embodiments, based on the novel and inventive techniques discussed herein, a system uses a semi-automatic algorithm to simulate contact between the teeth of both jaws or parts thereof (jaw portions). A graphical user interface (GUI) allows the user to partially specify the relative position and orientation of the jaws or parts thereof (jaw portions) using a limited set of clinically relevant spatial and rotational parameters. Any change the user makes to these parameters will be received by the algorithm. The algorithm then adjusts a different limited set of spatial and rotational parameters to meet certain optimization constraints, leading to an occlusion 108 that is presented to the user in the GUI.

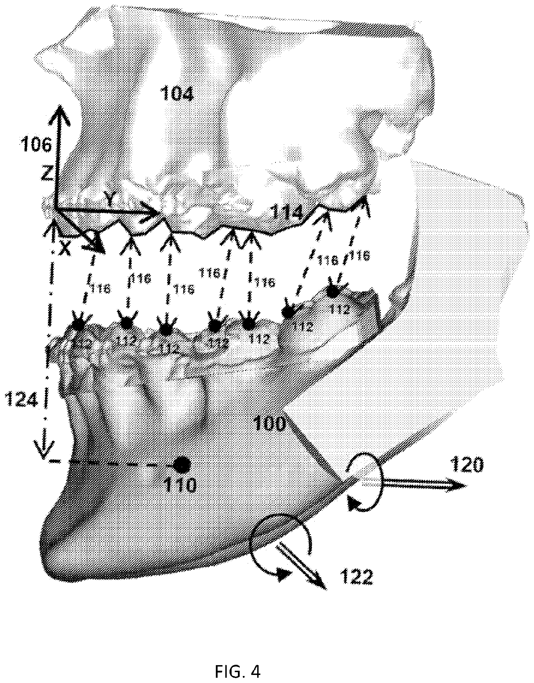

With reference to Figure 4 (and the defined coordinate systems of Figure 3), aspects of the present disclosure specifically allow user manipulation of the moving jaw portion 100 with respect to three position variables relative to the WCS 106. For example, the user controls the translation of the moving jaw portion 100 in the X-direction and the Y-direction, i.e., in the horizontal (X-Y) plane. The user also controls the rotation of the moving jaw portion 100 around the vertical (Z) axis. The user can also set the position of a control point 110. The control point 110 represents the pressure application point on the moving jaw portion 100.

For split mandible or split maxilla cases, the moving jaw portion can represent either one part of a jaw that results from splitting that jaw into multiple parts, or some or all parts of a jaw merged into a single object. The user may be able to switch between both representations, to alternate between manipulating the position and orientation of individual parts of a jaw, a group of jaw parts or the entire jaw.

Based on the user input controlling translation and rotation movement of the moving jaw portion 100 in the X-Y plane and setting the control point, the algorithm applies certain optimization parameters (described in detail below) and calculates a vertical translation, i.e., in the vertical (Z) direction. The algorithm also calculates rotations around the two horizontal (X and Y) axes to bring the teeth into optimized contact without modifying the parameters set by the user.

Accordingly, aspects of the present disclosure provide for a division of work: the algorithm automatically performs the otherwise tedious task of ensuring the teeth exactly make contact, while the user manipulates a more limited, but clinically relevant set of parameters. This saves time and allows for a greater focus on the clinical aspects of the occlusion 108 during the planning process. In addition, the parameters controlled by the user are defined in such a way that they are equivalent to well-known clinical measurements used to describe the occlusion 108, such as overjet and midline deviation, which simplifies cooperation between the surgeon and the clinical engineer using the planning software.

Mathematically (and as described above), the occlusion 108 can be represented as six degrees of freedom (DOF) of the moving jaw portion 100 with respect to the WCS 106 of the fixed jaw portion 104, three for rotation and three for translation. Thus, six separate values together uniquely determine the position and orientation of the jaws 100, 104 relative to one another.

As noted above, either the maxilla or mandible can be defined as the moving jaw portion 100, while the other jaw remains fixed and will be called the fixed jaw portion 104. As shown in Figure 3, the moving jaw portion 100 is expressed in the local coordinate system (LCS) 102, including a point in 3-D space acting as the origin and three orthonormal 3-D vectors representing the directions of the LCS X', Y' and Z' axes. The three spatial coordinates relative to the origin are the translation DOFs. The LCS axes are fully determined by a system of three Euler (Cardan) angles, which are the rotation DOFs.

For split mandible or split maxilla cases, to allow the user to manipulate the positions and orientations of individual parts of a split jaw, a part resulting from the splitting of either the maxilla or mandible can be defined as the moving jaw portion, while all parts of the other jaw remain fixed and will be called the fixed jaw portion.

The order of the rotations may be specified to resolve ambiguity. In certain embodiments, a Z-X-Y order of the rotations is applied. However, another order of the rotations may be applied instead. Similarly, whether the rotations are intrinsic (axes tied to rotating object) or extrinsic (axes remain stationary while object rotates) may be specified to resolve ambiguity. In certain embodiments, intrinsic rotations are applied. However, extrinsic rotations may be applied instead.

The LCS 102 is defined relative to the world coordinate system (WCS) 106 on the fixed jaw portion 104, which does not change. In the examples discussed herein, for the LCS 102 and the WCS 106 of the respective jaws 100, 104, the X-axis points left, the Y-axis points posteriorly, and the Z-axis points upward, while the origin is situated between the two middle incisor teeth. The directions of the WCS and LCS axes and the order of the rotations in the Euler angle system are arbitrary choices; the selection used here is not essential to the function of the embodiments discussed herein. However, it may be convenient to define the WCS and LCS as substantially parallel to each other when the fixed and moving jaw portions are in a relative position and orientation that can be considered neutral, e.g., in their relative position and orientation before the planned surgery as observed in pre-operative medical imaging. In typical use cases, rotation angle values will be low, so that the choice of sequence of Euler angles (X-Y-Z, Y-X-Z, etc.) and the choice between intrinsic and extrinsic rotations may not be noticeable to the user. Rotations are hereinafter expressed around the X, Y and Z axes, but the skilled person will readily appreciate that rotations may similarly be defined around the X', Y' and Z' axes, respectively. The naming conventions and definitions of coordinate system axes are not limiting for the methods and systems disclosed herein. For example, a method or system described herein with axes defined as: the X axis pointing left, the Y axis pointing towards posterior and the Z axis pointing upward, is, mutatis mutandis, entirely equivalent to a method or system with axes defined as: the X axis pointing right, the Y axis pointing towards anterior and the Z axis pointing upward, or to a method or system with axes defined as: the X axis pointing towards anterior, the Y axis pointing upward and the Z axis pointing right. Nonetheless, an unambiguous, and preferably clinically relevant definition may be chosen.

Rotations may be defined around the WCS origin or around the LCS origin. However, choosing the LCS origin as the center of rotation may lead to a more intuitive user experience. In the former case (rotations are defined around the WCS origin), to determine the LCS from three given rotation angles and three given translation distances, the LCS is first defined as coinciding with (having the same origin and axes as) the WCS; subsequently, the LCS is translated by a vector composed from the three translation distances; and finally, the LCS origin and axes are rotated in the WCS around the WCS origin using the rotation angles and the chosen sequence of Euler angles. In the latter case (rotations are defined around the LCS origin), to determine the LCS from three given rotation angles and three given translation distances, the LCS is first defined as coinciding with (having the same origin and axes as) the WCS; subsequently, the LCS axes are rotated around the WCS origin using the rotation angles and the chosen sequence of Euler angles; and finally, the LCS origin is translated in the WCS by a vector composed from the three translation distances.

As shown in Figure 3, for both the WCS 106 and the LCS 102, the origin is defined as the projection of the dental midline on the occlusal plane, i.e., a plane roughly passing through the cusps of the teeth. Aspects of the present disclosure contemplate that different WCS and LCS origins may be defined. However, this definition causes the X, Y and Z translations to correspond to measurements used in clinical practice, respectively the midline deviation, overjet and overbite. For example, an X translation of 0 mm will mean that the midline deviation is 0, or in other words that the mandible and maxilla midlines are aligned. Similarly, the rotations around the X, Y and Z axes will correspond with the values for respectively pitch, roll and yaw. These may be used to express the rotation of the moving jaw when communicating with a surgeon.

Table 1, below, sets forth the definition of the six DOFs that define an occlusion 108 and their distribution into three under user control and three under control of the algorithm as defined in certain embodiments of the present invention. Other embodiments may use different definitions of the six DOFs. However, the definition as listed in Table 1 has proven to be highly intuitive and to increase ease of use.

**TABLE 1**

| | **DOF name** | **Description** | **Controlled by** | **Clinical measurement** |
|---|---|---|---|---|
| **Translation** | X translation | Left-right displacement | User | Midline deviation |
| | Y translation | Anterior-posterior displacement | User | Overjet |
| | Z translation | Vertical displacement | Algorithm | Overbite |
| **Rotation** | X rotation | Sagittal plane rotation (around left-right axis) | Algorithm | Pitch |
| | Y rotation | Coronal plane rotation (around sagittal axis) | Algorithm | Roll |
| | Z rotation | Axial plane rotation (around vertical axis) | User | Yaw |

The DOFs under control of the user are considered fixed values for the semi-automatic algorithm, which only manipulates the remaining DOFs to simulate contact between both jaws. To fully define the occlusion, the algorithm finds a contact configuration between the fixed and moving jaw portion that constrains these remaining DOFs. In general, ignoring geometrical degeneracies, a frictionless (sliding) point contact between two smoothly shaped surfaces - such as the dental surfaces of the fixed and the moving jaw portion - removes (or, equivalently, constrains) one DOF from a mechanical system, as explained in Tasora, A., & Righettini, P. (2003), "Sliding Contact between Freeform Surfaces," Multibody System Dynamics 10, 239-262. Therefore, to fully define the occlusion, the algorithm may find a contact configuration comprising a number of contact points that is equal to the number of DOFs under the algorithm's control. For example, using the definitions from Table 1 above, the algorithm will only manipulate the values of three DOFs; therefore, it is sufficient for the algorithm to find three contact points.

For split mandible or split maxilla cases, if a split part contains the dental midline, it may be reasonable to apply the methods described above without changes, except that instead of the whole jaw, the part is considered the moving jaw portion. However, one or more parts of the split jaw may not include the dental midline. For example, if the maxilla is split into three parts, one of the lateral parts might contain only two premolars and two molars. In such cases, as noted above, it may be more convenient and may increase ease of use to locate the origin of the part's LCS in a point that is not the dental midline; instead, a point in the middle of the part could be used. Additionally, it may be more convenient to define the LCS so the X' axis is parallel to the direction of the row of teeth that the part comprises, tangential to the dental arc. The Y' axis may then be defined perpendicular to the X' axis and the Z' axis may then be defined perpendicular to both the X' axis and the Y' axis and substantially vertical. The X'-Y' plane will then be substantially parallel to the occlusal plane. Although the way in which the disclosed methods function does not materially change by this change in definition, the interpretation of the LCS positioning parameters as clinical measurements changes, as shown in Table 2 below:

**TABLE 2**

| | **DOF name** | **Description** | **Controlled by** | **Clinical measurement** |
|---|---|---|---|---|
| **Translation** | X translation | Displacement along dental arc | User | Mesialization / distalization |
| | Y translation | Displacement perpendicular to dental arc | User | Buccalization / lingualization |
| | Z translation | Vertical displacement | Algorithm | Intrusion / extrusion |
| **Rotation** | X rotation | Rotation around axis tangent to dental arc | Algorithm | Lingual / labial tipping |
| | Y rotation | Rotation around axis perpendicular to dental arc | Algorithm | Mesial / distal tipping |
| | Z rotation | Axial plane rotation (around vertical axis) | User | Yaw |

In some cases, it may be advantageous to bring an additional, fourth DOF under user control and reduce the number of DOFs controlled by the algorithm to two. In such cases, the algorithm may constrain these two DOFs by bringing the moving and stationary jaw portions into contact in fewer, e.g., two, contact points. For example, the X rotation may be put under direct control of the user instead of letting it be controlled by the algorithm, allowing the user additional control with the disadvantage of having to manipulate an additional variable. For example, in cases where the user is repositioning a full jaw, it may be necessary to have the teeth of both jaws make contact in only two points on the anterior side, at the incisor and/or canine teeth, and ensure there is still a gap between the teeth at the molars. After the planned orthognathic surgery, further orthodontic treatment or a bite increase can be used to close this gap. In such cases, control of the rotation around the WCS X axis, which represents rotation in the sagittal plane, can enable the user to ensure there is contact on the anterior side. In other cases, for example, in split mandible or split maxilla cases, where a part of the split jaw consists of several adjacent teeth that form an almost straight row resulting in the part having a very elongated shape, the contact surface between the part (which is treated as the moving jaw portion) and the stationary jaw portion will also have an elongated shape, behaving more like a line contact than an area contact. In such cases, it may not be possible to obtain a stable three-point contact and the X rotation (which represents a rotation around the WCS X axis, which is almost parallel to the line of contact) determined by the algorithm would behave in an erratic manner. A more stable solution could be obtained by giving the user control over this rotation instead of the algorithm, resulting in an occlusion in which the split jaw part makes contact with the stationary jaw portion in two points instead of three. Because the split jaw consists of multiple parts, two contact points per part would still result in four or more contact points for the full jaw, so the occlusion of the whole jaw is still sufficiently stable.

When the X rotation is under control of the user, based on the user input controlling the translation of the moving jaw portion 100 in the X-Y plane, the X rotation, the Z rotation and the location of the control point, the algorithm applies certain optimization parameters (described in detail below) and calculates a vertical translation, i.e., in the vertical (Z) direction. The algorithm also calculates a Y rotation acting around an approximately horizontal (Y) axis to bring the teeth into optimized contact without modifying the parameters set by the user.

As mentioned above, in some instances, instead of fixing the LCS origin to the projection of the dental midline on the occlusal plane, the user may instead be allowed to select a point that will act as the LCS origin for the moving jaw portion. This can be achieved for example by providing a graphical user interface (GUI) that allows the user to select a point on a 3D model representing the moving jaw portion. Although the relationship between the DOF values and the clinical measurements described above will be lost, this will allow the user to determine the center of rotation for the moving jaw portion, as the origin of the LCS is the point around which the moving jaw portion will appear to rotate when the DOFs representing rotations around the different axes are modified.

A method of determining a virtual occlusion is provided. The method includes obtaining a 3-D representation of a patient's first jaw portion and the patient's second jaw portion, the second jaw portion being opposite to and moveable with respect to the first jaw portion. The method further includes, with the 3-D representation, setting and representing in a graphic user interface (GUI) an initial position of the first jaw portion relative to the second jaw portion, the initial position being defined by a control point on the second jaw portion having six pre-determined degrees of freedom relative to a coordinate system with an origin fixed relative to the first jaw portion. The method further includes receiving user input of changes to at least one of the degrees of freedom. The user input of changes comprises a change to a position of the control point relative to the second jaw portion. The method further includes automatically adjusting at least one of the other degrees of freedom to minimize a distance, such as a vertical distance, of the control point to the origin while constraining distances between opposed surfaces of the first and second jaw portions to be positive, thereby determining an occlusion between the first and second jaw portions. The method further includes representing, in the GUI, the first and second jaw portions in the determined occlusion.

In some embodiments, the user input of changes comprises a change, such as a positive or negative change, to at least one of: a position of the second jaw portion in an X direction in the coordinate system corresponding to the patient's left direction, and the position of the second jaw portion in the Y direction in the coordinate system corresponding to the patient's posterior direction.

In some embodiments, the user input of changes comprises a change, such as a positive or negative change, to at least one of: a position of the second jaw portion in an X direction in the coordinate system corresponding to the patient's left direction, and the position of the second jaw portion in the Y direction in the coordinate system corresponding to the patient's posterior direction, an orientation of the second jaw portion around a Z direction in the coordinate system corresponding to the patient's upward direction.

In some embodiments, the user input of change to the position of the control point relative to the second jaw portion comprises change to the position of the control point in the Y' direction in the second jaw portion's local coordinate system. In some embodiments, the user input of change to the position of the control point relative to the second jaw portion comprises change to the position of the control point in one or more of the X', Y' and Z' directions in the second jaw portion's local coordinate system.

In some embodiments, the user input of changes comprises a change, such as a positive or negative change, to at least one of: a position of the second jaw portion in an X direction in the coordinate system corresponding to the patient's left direction, a rotation of the second jaw portion around the same X direction, the position of the second jaw portion in the Y direction in the coordinate system corresponding to the patient's posterior direction, and a rotation of the second jaw portion around a Z direction in the coordinate system corresponding to the patient's vertical direction.

In some embodiments , the user input of changes comprises a change, such as a positive or negative change, to at least one of: a position of the second jaw portion in an X direction in the coordinate system corresponding to a mesial-distal direction parallel to the dental arc, a rotation of the second jaw portion around the same X direction, the position of the second jaw portion in the Y direction in the coordinate system corresponding to a buccal-lingual direction perpendicular to the dental arc, and a rotation of the second jaw portion around a Z direction in the coordinate system corresponding to the patient's vertical direction.

In some embodiments, the user input of change to the position of the control point relative to the second jaw portion comprises change to the position of the control point in the X' direction in the second jaw portion's local coordinate system.

In some embodiments, the first jaw portion comprises the patient's maxilla, and the second jaw portion comprises the patient's mandible.

In some embodiments, the first jaw portion comprises the patient's mandible, and the second jaw portion comprises the patient's maxilla.

In some embodiments, the first jaw portion comprises the patient's maxilla, and the second jaw portion comprises a part of the patient's mandible resulting from a mandible split osteotomy.

In some embodiments, the first jaw portion comprises the patient's mandible, and the second jaw portion comprises a part of the patient's maxilla resulting from a maxilla split osteotomy.

In some embodiments, the first jaw portion comprises a part of the patient's maxilla resulting from a maxilla split osteotomy, and the second jaw portion comprises a part of the patient's mandible resulting from a mandible split osteotomy.

In some embodiments, the first jaw portion comprises a part of the patient's mandible resulting from a mandible split osteotomy, and the second jaw portion comprises a part of the patient's maxilla resulting from a maxilla split osteotomy.

In some embodiments, the GUI comprises a representation, such as a visualization of a 3-D model, of each of the first jaw portion and the second jaw portion.

In some embodiments, the GUI further comprises an occlusionogram of at least one of the first jaw portion and the second jaw portion.

In some embodiments, the GUI further comprises a display of the two or three contact points determined by the algorithm, for example showing dots on the locations of the contact points, superimposed on the 3-D models of the first and second jaw portions and / or on the occlusionogram.

In some embodiments, the GUI further comprises a display of additional approximate contact points, where an approximate contact point is defined as a point on the moving jaw portion for which the distance to a corresponding point on the fixed jaw portion is below a pre-determined threshold value - such as a threshold value between 0mm and 1mm, more specifically a threshold value of 0.1mm, 0.2mm, 0.3mm, 0.4mm, or 0.5mm - while both jaws are in occlusion. Displaying these points can give an indication of the area of the teeth that is almost in contact - and that could come into physical contact if there is some flexibility in the jaw. The approximate contact points can be displayed for example by showing dots on their locations, superimposed on the 3-D models of the first and second jaw portions and / or on the occlusionogram, e.g., with a different color than the color used for the two or three contact points determined by the algorithm. Optionally, only a selection of (approximate) contact points may be displayed. For example, the most relevant approximate contact points may be selected by projecting all approximate contact points onto the LCS X'-Y' plane, determining the two-dimensional convex hull of the projected points, and selecting only those approximate contact points for which the corresponding projected points are present on the boundary of the two-dimensional convex hull. To reduce visual clutter, only the selected approximate contact points may be shown; this will have the effect of eliminating a large number of points on the interior of the two-dimensional shape formed by the projected points while preserving the outer boundary, which shows the extent of the shape.

In some embodiments, the GUI further comprises a display of interactive user controls corresponding to the degrees of freedom controlled by the user. In some embodiments, these interactive user controls form part of the visualization of a 3-D model. In other embodiments, these interactive user controls form part of a separate window area of the GUI.

In some embodiments, the GUI further comprises a display of values corresponding to the degrees of freedom that are automatically adjusted.

In some embodiments, the GUI further comprises a display of controls that allow the user to switch between manipulating a single part resulting from the splitting of either the maxilla or mandible, and manipulating all parts of the entire jaw as a whole with the relative positions and orientations of these parts remaining fixed.

In some embodiments, while manipulating a single part resulting from the splitting of either the maxilla or mandible, the GUI further comprises the display of a control that enables or disables the visualization of the parts of the same jaw that are not being manipulated.

In some embodiments, the GUI further comprises the display of a control that determines whether the rotation around the WCS X axis is determined by the user or by the algorithm.

In some embodiments, the GUI further comprises a display of interactive user controls allowing the user to select a 3D point on the moving jaw portion to be used as the LCS origin.

In some embodiments, prior to determining the occlusion, the user may indicate a maximum allowable depth of burring on the occlusal surface of the 3-D models of one or both jaws. This can be achieved for example by letting the user select a maximum depth of burring, and letting the user apply a paintbrush-like tool to mark sections of the surface of the teeth corresponding to the region in which this maximum depth is to be applied. Many other ways of interactively indicating an area with a maximum allowable depth of burring are possible. For example, the user may use a lasso tool or a polygon lasso tool. Alternatively, the user may specify a line in the LCS or WCS X-Y plane. The areas of the surface of the teeth either anterior or posterior to a vertical plane (parallel to the Z axis) through this line could be used as the aforementioned region. Upon selection of this burring region, the maximum depth of burring may be applied by altering the 3-D model representing the affected jaw in such a way that the parts of the 3-D model representing the selected region, e.g., the points and triangles that the 3-D model's surface is made up of, are translated in a direction towards the inside of the model, thus generating a modified 3-D model. This direction can be the same for all points and triangles in the region, for example the vertical (Z) direction with downward translation for the mandible and upward translation for the maxilla. Alternatively, a surface offset with a negative distance value could be applied to the parts of the 3-D model representing the selected region. After generating one or more modified 3-D models in this way, they can be used instead of the corresponding original 3-D models for the semi-automatic virtual occlusion algorithm.

After the modified 3-D models representing the jaws have been generated to apply a maximum burring depth, the occlusionogram may be calculated in two different ways. Either version may be displayed to the user or both versions simultaneously. In certain aspects, both versions are calculated in the same way as when the 3-D models have not been manipulated but the 3-D models used as the input for the underlying distance calculation are different. The first version of the occlusionogram uses the modified 3-D models for those jaw parts for which modified 3-D models are available and the original 3-D models for all remaining jaw parts. Because the same 3-D models were used in the semi-automatic algorithm that simulates contact, the distances between both 3-D models that are shown on the occlusionogram will all be zero or greater than zero. These distances will quantify how much space there is between the teeth of both jaws when the teeth have been burred down to the indicated maximum burring depth over the entire region that the user indicated. The second version of the occlusionogram uses the original 3-D models for all jaw parts, as they were before the manipulation to apply the maximum burring depth took place, but in the relative position and orientation determined by applying the semi-automatic algorithm that simulates contact to the modified 3-D models. By consequence, the distances between both 3-D models that are shown on the occlusionogram may be smaller than zero in certain parts of the occlusal surface of the teeth, indicating interpenetration between the 3-D models of both jaws in those parts. The absolute values of these negative distances indicate the actual burr depth that would be required to be able to obtain the occlusion that has been calculated by the contact simulation algorithm.

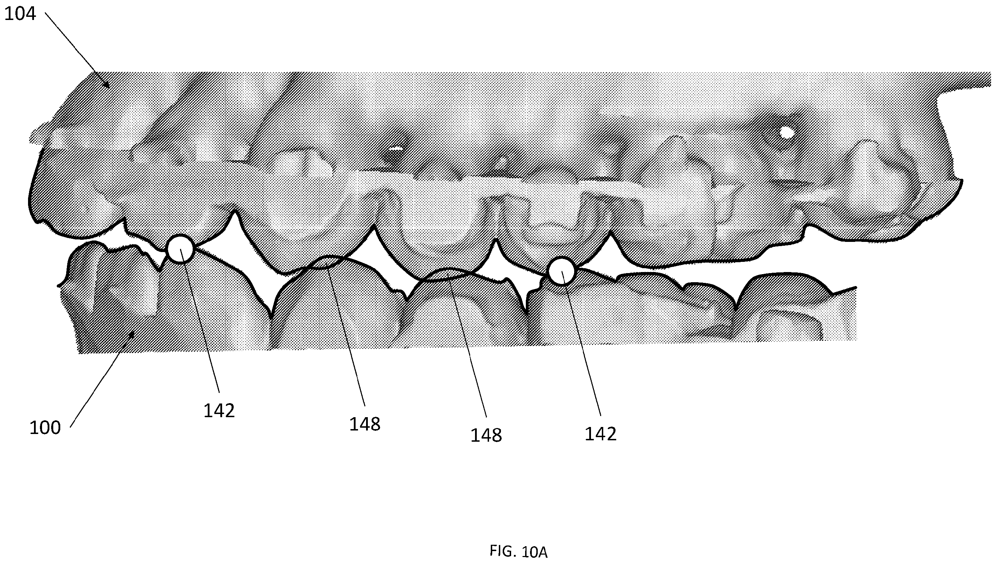

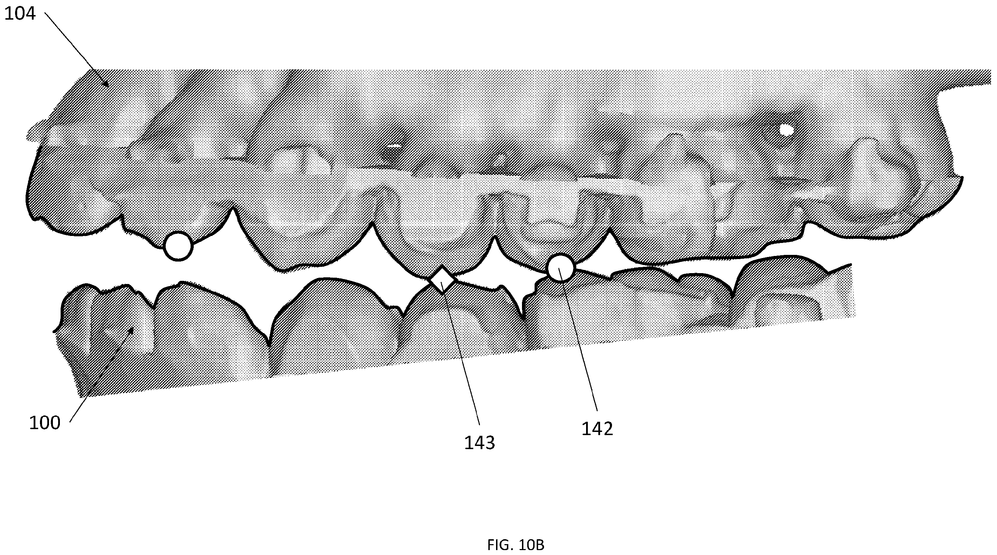

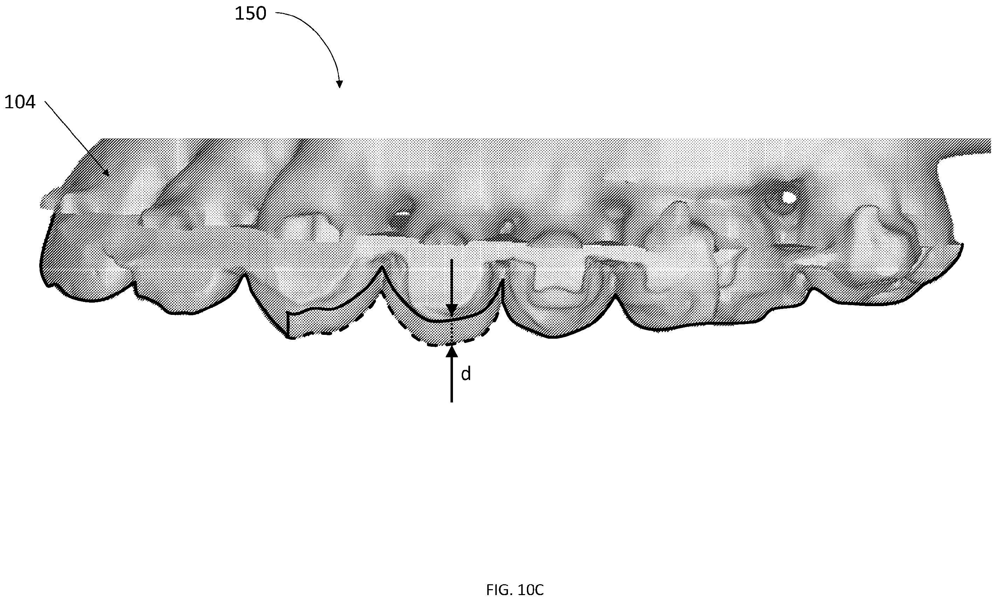

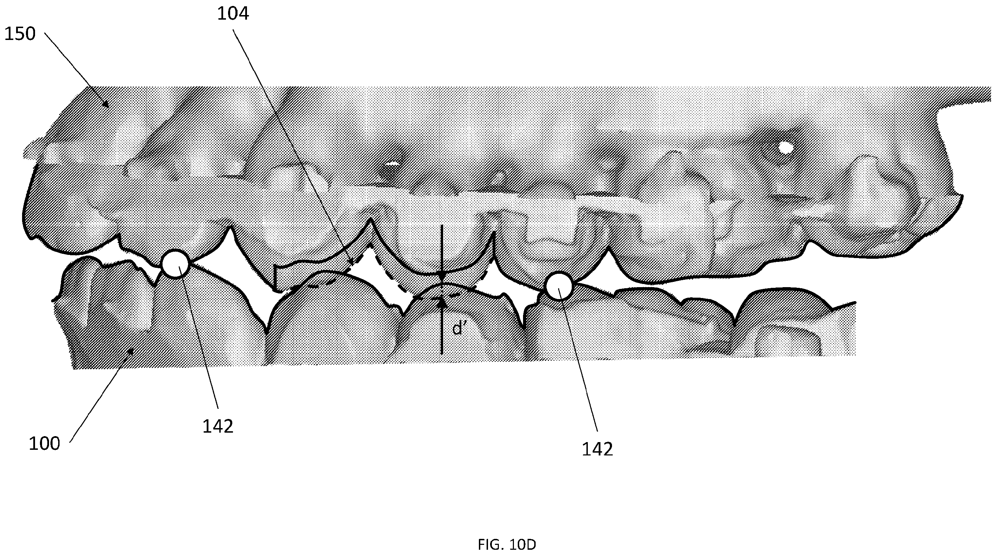

Figures 10A-D illustrate a case in which local burring may be desirable. Figure 10A shows a lateral view of the 3-D models representing maxilla 104 and mandible 100, with occlusal surfaces indicated as solid lines. The maxilla and mandible 3-D models are shown in a desired occlusion with contact in two points 142, indicated as dots. However, in this desired occlusion, the occlusal surfaces of both jaws interfere in two locations 148. The user may desire to remove this interference by removing part of the teeth of one or both jaws by burring. Without modification to the 3-D models, the methods disclosed herein may determine an occlusion as shown in Figure 10B, which includes contact in an undesired location 143, indicated as a diamond. To avoid this situation the user may indicate an area with a maximum burring depth. As depicted in Figure 10C, a modified 3-D model 150 may then be generated, e.g., based on the original 3-D model of maxilla 104, by applying an inward offset over the maximum burring depth d to the indicated area, shown as a dashed line. The resulting modified 3-D model 150, shown as a solid line, may then be used to determine occlusion. Figure 10D shows the resulting occlusion. This result matches the desired occlusion: the mandible 3-D model 100 makes contact with the modified maxilla 3-D model 150 in the two desired contact points 142, without interference in other locations. The original maxilla 3-D model 104 (dashed line), in the same occlusal pose, does interfere with the mandible 100. The negative distance d' between both models indicates the actual depth of burring required to obtain this occlusion.

In some embodiments, the method further includes updating the representation of the first and second jaw portions in the GUI in the occlusion determined in response to additional user input of changes to at least one of the degrees of freedom and corresponding automatic adjustments of at least one of the other degrees of freedom.

In some embodiments, a method of forming a surgical guide based on a determined virtual occlusion is provided. The method may include obtaining a 3-D representation of a patient's first jaw portion and the patient's second jaw portion, the second jaw portion being opposite to and moveable with respect to the first jaw portion. The method may further include, with the 3-D representation, setting an initial position of the first jaw portion relative to the second jaw portion, the initial position being defined by a control point on the second jaw portion having six pre-determined degrees of freedom relative to a coordinate system with an origin fixed relative to the first jaw portion. The method may further include receiving user input of changes to at least one of the degrees of freedom. The method may further include automatically adjusting at least one of the other degrees of freedom to minimize a vertical distance of the control point to the origin while constraining distances between opposed surfaces of the first and second jaw portions to be positive, thereby determining an occlusion between the first and second jaw portions. The method may further include forming a customized surgical guide to guide the patient's teeth into the determined occlusion during orthognathic surgery.

In some embodiments, the method further comprises representing, in a graphic user interface (GUI), the first and second jaw portions in the determined occlusion.

Disclosed herein are also systems for carrying out any of the methods disclosed herein.

Disclosed herein are also non-transitory computer-readable storage media, having instructions stored therein, which, when executed by a computing device, causes the computing device to carry out any of the methods disclosed herein.

Disclosed herein are also computer programs comprising instructions which, when executed by a computing device, cause the computing device to carry out any of the methods disclosed herein.

The following description and the related drawings set forth in detail certain illustrative features of one or more embodiments.

### BRIEF DESCRIPTION OF THE DRAWINGS

The appended figures depict certain aspects of the one or more embodiments and are therefore not to be considered limiting of the scope of this disclosure.
Figure 1 depicts an example of a conventional workflow of determining a desired occlusion for orthognathic surgery planning;
Figure 2 depicts an example of another conventional workflow of determining a desired occlusion for orthognathic surgery planning;
Figure 3 depicts an example of defined coordinate systems of a moving jaw and a stationary jaw;
Figure 4 depicts an example of a moving jaw and a stationary jaw with various points used for optimizing occlusion;
Figure 5 depicts an example of a graphic user interface (GUI) for determining and displaying a virtual occlusion;
Figure 6 depicts an example of a detail of the GUI of Figure 5;
Figure 7A depicts an example of a generalized diagram of a semi-automatic method of determining a virtual occlusion;
Figure 7B depicts an example of a block diagram of a schematic overview of a system for determining a virtual occlusion;
Figure 7C depicts an example of a block diagram of a schematic overview of a system for determining a virtual occlusion;
Figure 8 depicts an example of a sequence of steps for determining a virtual occlusion;
Figure 9 depicts an example of a moving jaw and a stationary jaw with various points used for optimizing occlusion with an example spit maxilla;
Figures 10A-D depict an example of local burring for obtaining desired occlusion;
Figures 11A and 11B depict a close-up of an occlusionogram of a maxilla; and
Figure 12 illustrate an example 3-D shape of a surgical guide based on a determined occlusion.

### DRAWING REFERENCE NUMERALS

2 create impression molds of teeth
4 create plaster casts from impression molds
6 position plaster casts in articulator to determine and represent desired occlusion
8 fix plaster casts together in desired occlusion
10 scan fixed plaster casts for planning
22A separately scan plaster casts
22B intra-oral scan of both jaws
24 virtually determine desired occlusion and specify relative position and orientation of jaws relative to one another
26 depict jaws in desired occlusion for planning
100 moving jaw
1001, 1002, 1003 moving split jaw part
102, 1021, 1022, 1023 local (moving) coordinate system
104 fixed (or stationary) jaw
106, 1061, 1062, 1063 world (fixed) coordinate system
108 occlusion (mathematically defined as position (in 6 DOF) of LCS in WCS)
110 control point
112 collision vertices on moving jaw
114 collision triangles (2D) on fixed jaw
116 distances *dᵢ* between 112 and 114, constrained as positive
118 vertical (Z, overbite) translation variable
120 coronal plane (Y, roll) rotation variable
122 sagittal plane (X, pitch) rotation variable
124 control point Z coordinate of 110, minimized subject to 116 constraint 126 user controls
128 midline (X) translation variable
130 overjet (Y) translation variable
132 axial plane (Z, yaw) rotation variable
134 3-D model of jaws
136 occlusionogram of fixed jaw
138 occlusionogram of moving jaw
140 pressure distance (control point Y) variable
142, 143 contact point
144 approximate contact point
146 convex hull
148 interference
150 modified 3-D model
1200 surgical guide
1202 guide body
1204 top surface
1206 bottom surface
1208 edge
1210 indentation

To facilitate understanding, identical reference numerals have been used, where possible, to designate identical elements that are common to the drawings. It is contemplated that elements and features of one embodiment may be beneficially incorporated in other embodiments without further recitation.

### DETAILED DESCRIPTION

Aspects of the present disclosure provide apparatuses, methods, processing systems, and computer readable mediums for determining virtual occlusion for orthognathic surgery planning. The embodiments described herein resolve several problems with conventional systems and methods.

The embodiments disclosed herein generally follow the coordinate system definition of Table 1 for use cases where the moving jaw portion comprises the middle incisors and the coordinate system definition of Table 2 for use cases where the moving jaw portion does not comprise the middle incisors. It should be noted, however, that the proper functioning of the systems and methods disclosed herein does not rely on a particular definition or naming convention of the world or local coordinate systems. In certain aspects, aligning a coordinate system axis with a clinically relevant direction or positioning a coordinate system origin in a clinically relevant location may make the operation of the systems and methods disclosed herein more intuitive, convenient or user friendly. However, to benefit from these advantages, it is not critical which axis in particular is aligned with such a clinically relevant direction. Statements that focus on a particular axis are meant to reflect the clinically relevant direction rather than the axis. For example, embodiments disclosed herein may be described based on a coordinate system definition that aligns the X axis with the patient's left/right direction, the Y axis with the patient's posterior/anterior direction and the Z axis with the patient's superior/inferior direction. In this example, a statement such as "movement is restricted along the Y axis" is meant to be understood as "movement is restricted along the posterior/anterior direction". An implementation using a different naming convention for the axes but paying suitable attention to clinically relevant locations and directions will yield similar or the same benefits.

Embodiments of the present disclosure enable semi-automatic control of the occlusion 108 by dividing the six DOFs into three DOFs that are under user control, such as through a GUI or other suitable interface, and three that are manipulated by a semi-automatic algorithm. Specifically, the user may control the translation in a horizontal plane (along X and Y axes) and the rotation around a vertical (Z) axis. The algorithm does not alter these but calculates the vertical translation (along the Z axis) and the rotation around two horizontal (X and Y) axes to bring the teeth into three-point contact. In addition, the user controls the location of a control point 110 on the moving jaw portion 100 that will indirectly influence the algorithm. The control point 110 represents a pressure application point on the moving jaw portion 100.

This division of the six DOFs into user-controlled parameters and automatically-controlled parameters can be compared to physically positioning an object on an uneven, generally horizontal surface: the translations in the horizontal plane (X and Y) and the rotation around the vertical axis (Z) determine where on the surface the object is placed and in what direction it is facing, which is controlled by the user. The other three DOFs (vertical translation and rotation around X and Y) will be determined by how the object moves under gravity until it comes to rest on three contact points. In this comparison, the control point 110 represents the center of gravity, or the point on which one presses down on the object. Moving the control point 110 far enough will cause the object to tip over until it comes to rest on a different set of three contact points. The user may control the position of the control point 110. In some embodiments, the user only controls the position of the control point 110 in the Y direction, e.g., antero-posteriorly back and forth using the coordinate system definition of Table 1. In some embodiments, the user only controls the position of the control point 110 in the X' direction, e.g., the mesial/distal direction using the coordinate system definition of Table 2. However, alternative embodiments may provide for user control of the position of the control point 110 in all directions.

According to certain embodiments, the semi-automatic algorithm that simulates contact between the teeth is an iterative optimization algorithm. The optimization algorithm manipulates three variables (the three DOFs not under user control) to maximize the vertical displacement (Z direction in WCS 106) of the control point 110 on the moving jaw portion 100 in order to close the jaws 100, 104 together. For example, in the case of the mandible set as the moving jaw portion 100, maximizing the Z position will push the jaws 100, 104 together. In the case of the maxilla set as the moving jaw portion 100, minimizing the Z position will push the jaws 100, 104 together. In addition, the optimization algorithm is constrained by collision between the moving and fixed jaw portions. This is achieved by prohibiting some or all of the surfaces of moving jaw portion 100 to pass through some or all of the surfaces of fixed jaw portion 104. For example, in embodiments in which both jaw portions are represented by triangle meshes, the optimization algorithm may be constrained so that collision points 112 defined on the surface of the moving jaw portion 100 are not allowed to pass through collision triangles 114 defined on the surface of the fixed jaw portion 104. Alternatively, collision points 112 may be defined on the surface of the fixed jaw portion 104 and collision triangles 114 may be defined on the surface of the moving jaw portion 100. Because the optimization algorithm controls exactly three DOF variables, and in general (ignoring degenerate cases) each point-triangle pair constrains a single DOF and therefore exactly three point-triangle pairs will constrain the solution, the outcome is unique and fully determined by the parameters under user control (three DOFs and the position of the control point 110). In order to avoid degenerate solutions, e.g., jaws 100, 104 being positioned so far apart that no collision triangles 114 can be found to constrain the solution, some embodiments may prevent the movable jaw portion 100 from being moved too far so as to result in a degenerate solution. It should be noted that the use of triangle meshes is not mandatory. Other representations of 3D shape data of the moving and fixed jaw portions may be utilized in certain aspects. For example, both jaw portions may be represented using other types of surface elements, such as freeform surface elements. The optimization algorithm will then determine three surface-element pairs.

As discussed, though certain aspects are described with three DOFs that are under user control and three that are manipulated by a semi-automatic algorithm, it should be noted that in some aspects, similarly and as discussed, four DOFs may be under user control and two may be manipulated by a semi-automatic algorithm. For example, other embodiments of the present disclosure enable an alternative semi-automatic control of the occlusion by dividing the six DOFs into four DOFs that are under user control, such as through a GUI or other suitable interface, and two that are manipulated by a semi-automatic algorithm. Specifically, the user may control the translation in a horizontal plane (along X and Y axes) and the rotations around a vertical (Z) axis and a horizontal (X) axis. The algorithm does not alter these but calculates the vertical translation (along the Z axis) and the rotation around the other horizontal (Y) axis to bring the teeth into two-point contact. In addition, the user controls the location of a control point on the moving jaw portion that will indirectly influence the algorithm. The control point represents a pressure application point on the moving jaw portion.

In this alternative semi-automatic control method, the effect of the control point is similar to its effect in the three-point contact algorithm described previously, with the exception that the moving jaw portion will make contact with the stationary jaw portion in two contact points rather than three, and the control point determines at which pair (rather than triplet) of points contact will occur. Because the rotation around the X axis (left-right according to the coordinate system definition of Table 1 or tangential to dental arc according to the coordinate system definition of Table 2) is locked in by the user, the position of the control point will have no influence on it. By consequence, the position of the control point in the Y direction (anterior-posterior or perpendicular to dental arc) has no influence on the algorithm, and it will suffice to let the user only control the position of the control point in the X direction (left-right or mesial-distal, respectively).

According to such embodiments, and similar to the embodiments described above, the semi-automatic algorithm that simulates contact between the teeth is an iterative optimization algorithm. The optimization algorithm manipulates two variables (the two DOFs not under user control) to maximize the vertical displacement (Z direction in WCS) of the control point on the moving jaw portion in order to close the jaws together. For example, in the case of the mandible set as the moving jaw portion, maximizing the Z position will push the jaws together. In the case of the maxilla set as the moving jaw portion minimizing the Z position will push the jaws together. In addition, the optimization algorithm is constrained by collision between the moving and fixed jaw portions. This is achieved by prohibiting some or all of the surfaces of moving jaw portion 100 to pass through some or all of the surfaces of fixed jaw portion 104. For example, in embodiments in which both jaw portions are represented by triangle meshes, the optimization algorithm may be constrained so that collision points defined on the surface of the moving jaw portion are not allowed to pass through collision triangles defined on the surface of the fixed jaw portion. Alternatively, collision points 112 may be defined on the surface of the fixed jaw portion 104 and collision triangles 114 may be defined on the surface of the moving jaw portion 100. Because the optimization algorithm controls exactly two DOF variables, and in general (ignoring degenerate cases) each point-triangle pair constrains a single DOF and therefore exactly two point-triangle pairs will constrain the solution, the outcome is unique and fully determined by the parameters under user control (four DOFs and the position of the control point). In order to avoid degenerate solutions, e.g., jaws being positioned so far apart that no collision triangles can be found to constrain the solution, some embodiments may prevent the movable jaw portion from being moved too far so as to result in a degenerate solution. As with the other embodiments, it should be noted that the use of triangle meshes is not mandatory. Other representations of 3D shape data of the moving and fixed jaw portions may be utilized in certain aspects. For example, both jaw portions may be represented using other types of surface elements, such as freeform surface elements. The optimization algorithm will then determine two surface-element pairs.

Many well-known optimization techniques can be used to implement the embodiments discussed herein, such as gradient descent methods, the Nelder-Mead method (Gao and Han 2012), the Powell method (Powell 1964) or even brute-force search. As long as the optimization algorithm yields a correct result within the constraints described herein, the choice of the optimization algorithm only influences the running time of the algorithm and, by consequence, the responsiveness of the GUI.

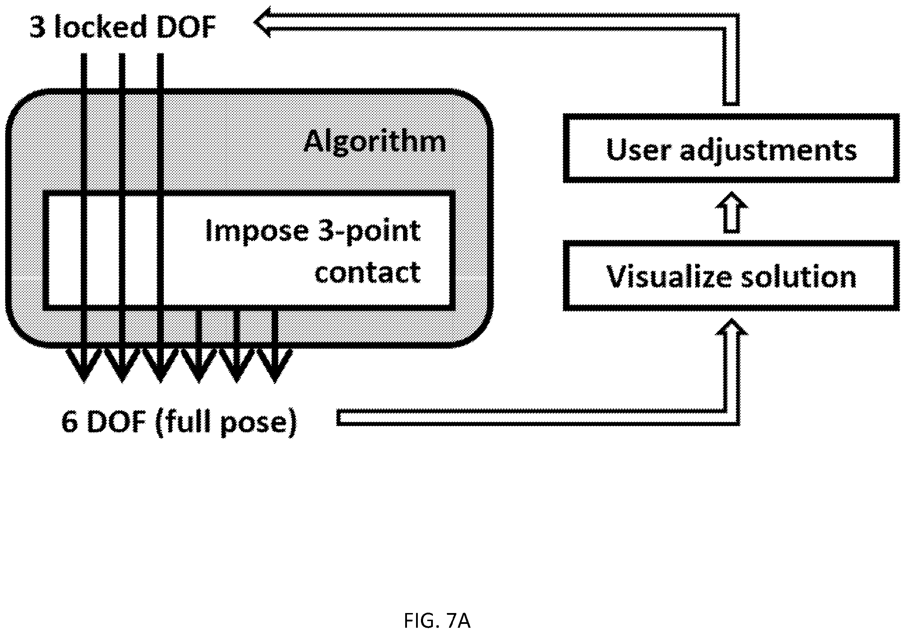

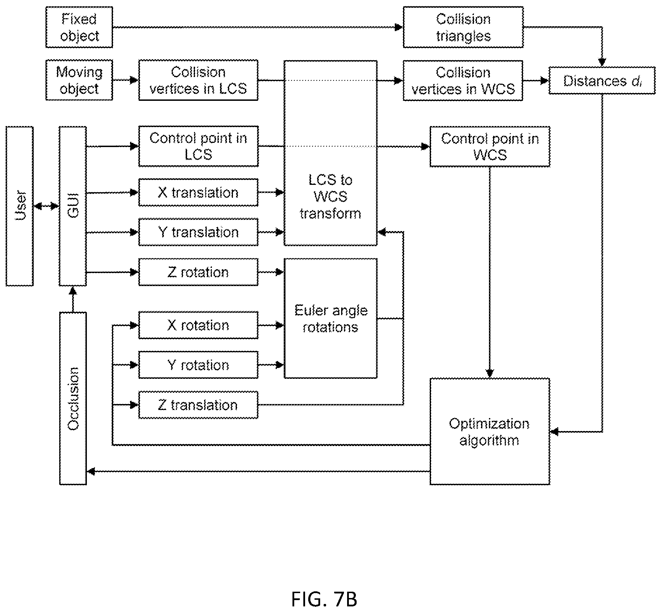

Regardless of the optimization technique selected, the contact simulation is specified as an optimization problem. As can be seen in Figure 4 (and Figures 7A, 7B), the algorithm resolves the optimization problem as follows.

First, the optimization variables are the three DOFs under control of the algorithm (X rotation, Y rotation and Z translation, in the WCS 106).

Second, the goal function minimized is the Z coordinate of the control point 110 that is defined on the moving jaw portion 100. If the mandible is the moving jaw portion 100, the negative Z coordinate is used, so minimizing the value of the goal function maximizes the vertical displacement of the control point from its initial position in a direction that closes the jaw. An optimization function may be either a minimizer or maximizer. According to certain embodiments, a minimizing optimization function is used. The same optimization function could alternatively yield a maximum value, by multiplying by -1. If the mandible is the moving jaw portion 100, the Z position of the control point 110 may be maximized, and in such a case, the negative of the Z position can be the optimization metric because minimizing -Z corresponds to maximizing Z.

Third, the optimization is subject to a set of inequality constraints *dᵢ* ≥ 0, one for each collision point, where *dᵢ* is the signed distance 116 from the collision point 112 (on the moving jaw portion 100) indexed by i to the closest point on any of the collision triangles 114 (on the fixed jaw portion 104), with the sign positive if the collision point is outside the fixed jaw portion 104. As described above, alternatively the collision points 112 may be defined on the fixed jaw portion 104 and the collision triangles 114 may be defined on the moving jaw portion 100.

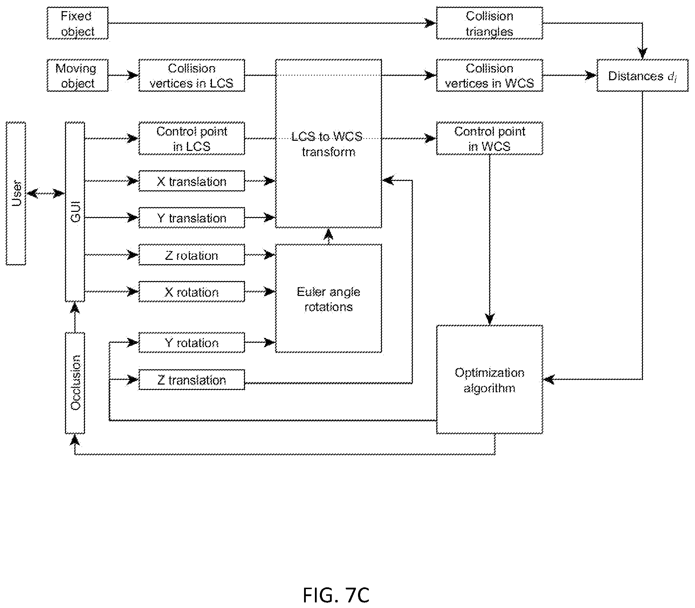

Similarly, as illustrated in Figure 7C, in those embodiments where the user is able to manipulate four DOFs, including the rotation around the X axis, the contact simulation is specified as an optimization problem.

First, the optimization variables are the two DOFs under control of the algorithm (Y rotation and Z translation, in the WCS 106).

Second, the goal function minimized is the Z coordinate of the control point 110 that is defined on the moving jaw portion 100. If the mandible is the moving jaw portion 100, the negative Z coordinate is used, so minimizing the value of the goal function maximizes the vertical displacement of the control point from its initial position in a direction that closes the jaw. An optimization function may be either a minimizer or maximizer. According to certain embodiments, a minimizing optimization function is used. The same optimization function could alternatively yield a maximum value, by multiplying by -1. If the mandible is the moving jaw portion 100, the Z position of the control point 110 may be maximized, and in such a case, the negative of the Z position can be the optimization metric because minimizing -Z corresponds to maximizing Z.

Third, the optimization is subject to a set of inequality constraints *dᵢ* ≥ 0, one for each collision point, where *dᵢ* is the signed distance 116 from the collision point 112 (on the moving jaw portion 100) indexed by i to the closest point on any of the collision triangles 114 (on the fixed jaw portion 104), with the sign positive if the collision point is outside the fixed jaw portion 104. As described above, alternatively the collision points 112 may be defined on the fixed jaw portion 104 and the collision triangles 114 may be defined on the moving jaw portion 100.

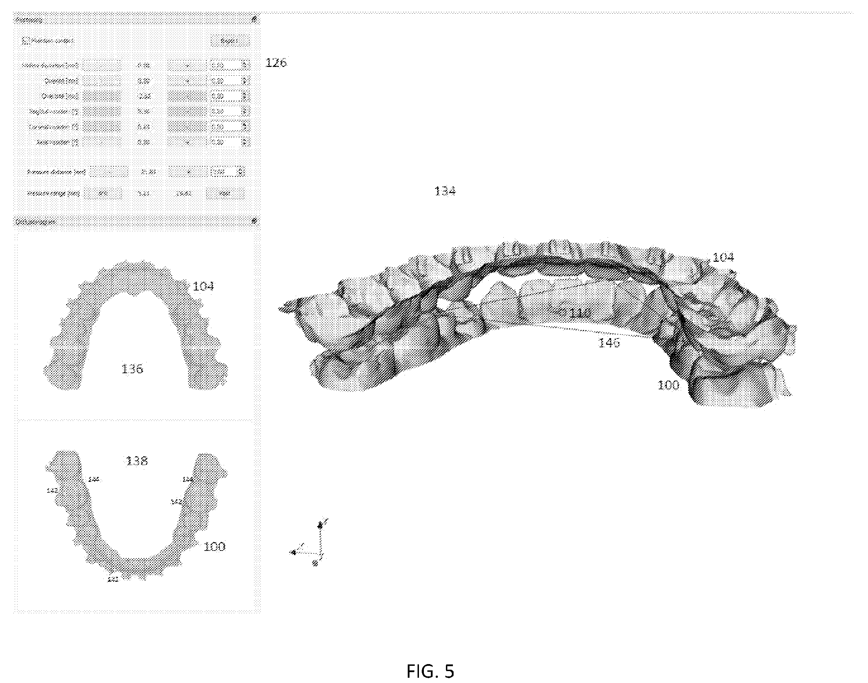

An example screenshot of the GUI according to aspects of the present disclosure is shown in Figure 5. The GUI performs, for example, two functions: 1) it allows the user to modify the three or four user-controlled DOFs as well as the control point, and 2) it presents the occlusion calculated by the semi-automatic algorithm to the user.

In the example shown in Figure 5, the GUI provides a simple set of buttons 126 that increase or decrease the value of each DOF by a certain amount (itself specified using a spin box GUI element). According to an embodiment, the control point 110 is specified by its Y coordinate-which is controlled in the same manner as the other 3 DOFs-while the X and Z coordinates remain zero.

The resulting occlusion calculated by the algorithm is presented to the user, via the GUI, as a 3-D model 134 of both jaws 100, 104 as well as two occlusionograms 136, 138. Occlusionogram 136 shows a bottom-up view of the maxilla (in the examples shown and described, fixed jaw portion 104). Occlusionogram 138 shows a top-down view of the mandible (in the examples shown and described, moving jaw portion 100). Although not shown in Figure 5, occlusionograms 136 and/or 138 may show the vertical distances between the teeth as a color plot superimposed on the view of the respective jaw portion 100, 104. Alternatively or additionally, the distances may be displayed as a color map superimposed onto the 3-D model. It may be convenient for the GUI to offer the possibility to selectively show or hide individual jaw parts in the 3-D model. The GUI may also visualize the contact points 142 and/or approximate contact points 144, e.g., by means of dots or diamonds, on the occlusionogram and/or the 3-D model. The GUI may further visualize the convex hull 146 around the contact points 142 and/or approximate contact points 144. For two-point contact, the convex hull 146 around the contact points 142 will be a line segment connecting the two contact points. For three-point contact, the convex hull 146 will be a triangle connecting the three contact points 142. The convex hull around approximate contact points 144 may be calculated as described above and may also be visualized, e.g., as a polygon. The GUI may offer the user the possibility to choose which convex hull 146 should be visualized, if any. Visualizing the convex hull 146 may offer the user additional insight into the general stability of the occlusion. The GUI may further offer the user the possibility to indicate areas with a maximum burring depth as described above. The GUI may then further offer the user the possibility to choose between an occlusionogram based on the modified 3-D models with the maximum burring depth applied or based on the original 3-D models, as described above. Alternatively, the GUI may display both types of occlusionograms simultaneously. The GUI may further offer the user the possibility to indicate areas in which the contact points should lie, or areas in which contact points should not be present, as described below. The specific implementation of these functions is not essential to the embodiments described herein, it only matters that the three or four DOF values and the control point location are unambiguously specified by the user and that the resulting occlusion 108 is presented to the user.

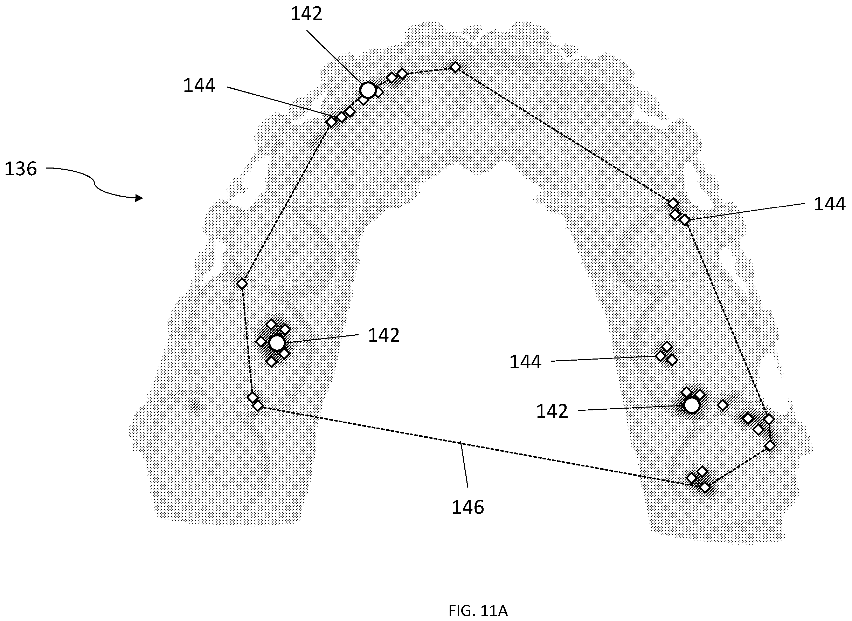

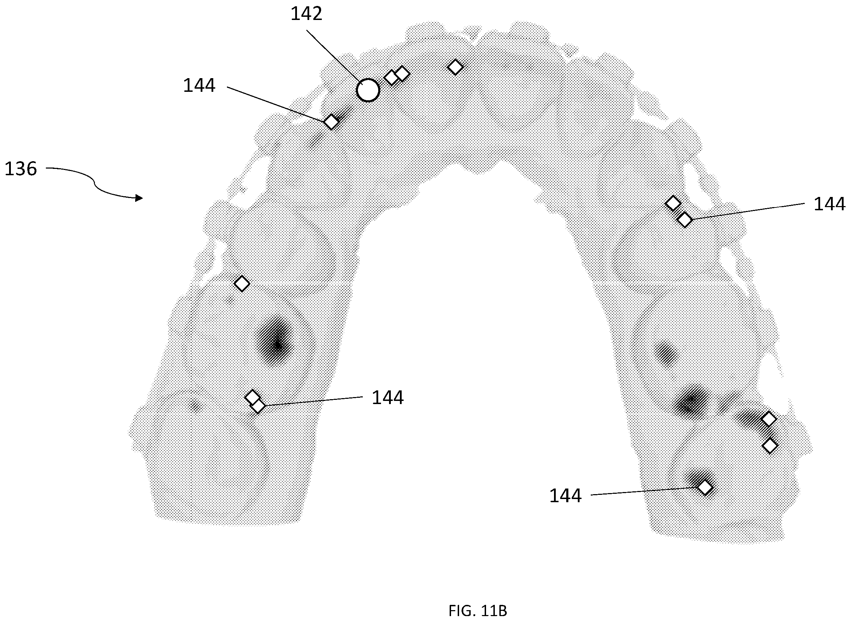

Figures 11A and 11B show a close-up of an occlusionogram 136 of a maxilla 104. Darker colors indicate closer distances from the maxilla's occlusal surface to the mandible's occlusal surface. In Figure 11A, the three contact points 142, indicated by dots, determined by the semi-automatic contact simulation algorithm, for which the distance between maxilla and mandible is zero, are displayed. Additional approximate contact points 144, indicated by diamonds, for which this distance is below a threshold value are also shown, along with the convex hull 146, indicated as a dashed line, of all the approximate contact points. Figure 11B shows the same occlusionogram 136, but only shows the contact points 142 (only one in this example) and approximate contact points 144 that lie on the convex hull, thus reducing visual clutter.

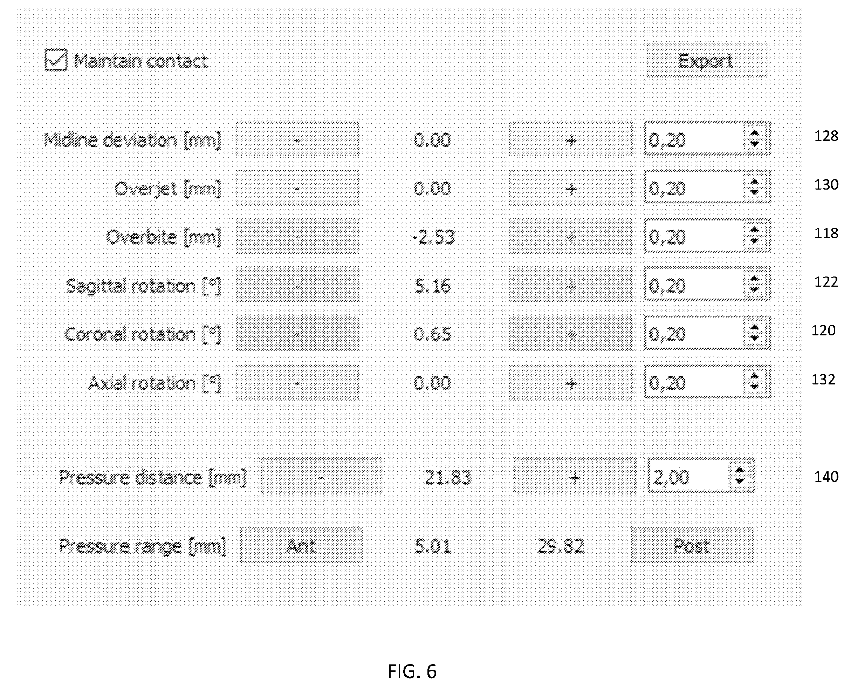

Figure 6 is a detail of the buttons 126 of an example of the GUI shown in Figure 5. Using the GUI of this example, the user can adjust the midline deviation, i.e., translation in the X direction, with buttons 128. Similarly, the user can adjust the overjet, i.e., translation in the Y direction, with buttons 130. Likewise, the user can adjust rotation in the axial plane, i.e., rotation about the Z axis, with buttons 132. Further, the user can adjust the position of the pressure point 110 in the Y direction with buttons 140. The GUI also shows the parameters that are automatically calculated, so that the user and/or the surgeon is presented with the detailed position information in real-time. The automatically calculated overbite, i.e., translation in the Z direction, is shown in window 118. The automatically calculated coronal plane rotation, i.e., rotation about the Y axis or roll, is shown in window 120. The automatically calculated sagittal plane rotation, i.e., rotation about the X axis or pitch, is shown in window 122. Presenting the DOF values in the GUI using clinically relevant captions may make the GUI more intuitive, convenient and user friendly. As can be seen in Figure 6, buttons 118, 120, 122 for the three automatically calculated DOF values are disabled. In those embodiments where the user can control an additional, fourth, DOF value, e.g., sagittal rotation, the corresponding buttons are not disabled.

In addition to the set of buttons 126 or as an alternative to it, the GUI may offer interactive controls for the three or four user-controlled DOF values superimposed onto 3-D model 134, such as straight arrows for translations and curved arrows for rotations.

The block diagram in Figure 7A is a generalized diagram of the semi-automatic method according to embodiments described herein. The block diagram in Figure 7B provides a schematic overview of a system for determining the virtual occlusion 108 according to an example embodiment. Through the GUI, the user determines the values of the three user-controlled DOFs-X translation 128, Y translation 130, and Z rotation 132-and Y position of the control point 110 defined in the moving jaw's LCS 102. The three other DOFs-Z translation 118, X rotation 122, and Y rotation 120-are automatically calculated by the algorithm, subject to the user controlled DOFs and the optimization with constraints described herein. The person skilled in the art will readily understand that the block diagrams in Figure 7A and Figure 7B also apply, mutatis mutandis, to embodiments in which the user can control a different set of three DOFs or, as is illustrated in Figure 7C, in which the user can control four DOFs.

The rotation DOFs are used to define a set of Euler angle rotations which, along with the translation DOFs, define the mathematical transformation from LCS 102 to WCS 106. This transformation is applied to both the collision vertices 112 and the control point 110 (both defined on the moving object) to obtain the corresponding WCS coordinates.

The distances 116 (*dᵢ*) between collision vertices 112 and collision triangles 116 are calculated and act as constraints in the optimization algorithm, while the WCS Z coordinate of the control point 110 is the optimization goal function. Based on these, the optimization algorithm defines new estimates for the DOFs it controls, iterating the previous steps until convergence is reached. Finally, the resulting DOFs are used to define the occlusion, which is presented to the user in the GUI, who can then evaluate the result and modify the user-controlled parameters accordingly.

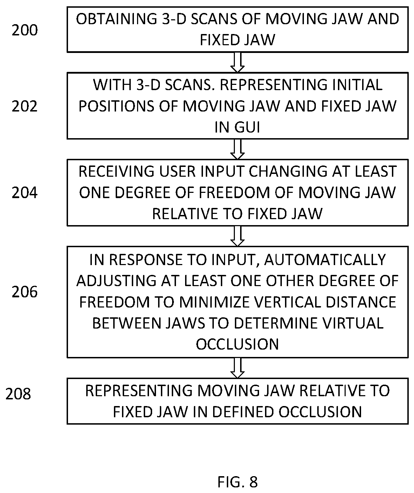

Figure 8 is an example flow chart setting forth a sequence of steps according to certain embodiments. In step 200, 3-D representations of a patient's moving jaw portion 100 and fixed jaw portion 104 are obtained. According to certain embodiments, the 3-D representations may be optical surface scans, e.g., optical surface scans of plaster casts or intra-oral scans. Alternatively, the 3-D representations may be derived from medical images, such as CT, MRI or CBCT images. The moving jaw portion 100 may be the patient's mandible, and the fixed jaw portion 104 may be the patient's maxilla. Alternatively, the moving jaw portion 100 may be the patient's maxilla, and the fixed jaw portion 104 may be the patient's mandible. One or more of the fixed and moving jaw portions may be split jaw parts.

In step 202, the 3-D representations are used to represent an initial position of the moving jaw portion 100 relative to the fixed jaw portion 104 in a GUI. The initial position may be defined by the control point 110 on the moving jaw portion 100 having six pre-determined degrees of freedom relative to the WCS 106 with an origin fixed relative to the fixed jaw portion 104. The WCS 106 may have the origin at the midline of the middle incisors of the fixed jaw portion 104. The WCS 106 may have an X axis with positive values in the patient's left direction. The WCS 106 may have a Y axis with positive values in the patient's posterior direction. The WCS 106 may have a Z axis with positive values in a vertical, up direction. The control point 110 may be positioned on the Y axis. Alternatively, e.g., when the moving jaw portion does not comprise the middle incisors, the X axis may be tangential to the dental arc with positive values in the distal direction, the Y axis may be perpendicular to the X axis with positive values in the lingual direction and the Z axis may be vertical, with positive values in the up direction. The skilled person will readily appreciate that other definitions may be chosen, as mentioned above. In certain embodiments, step 202 may further include indicating an area with a maximum burring depth and generating at least one modified 3-D representation 150 of at least one jaw portion by applying an inner offset over a distance equal to the maximum burring depth to the indicated area.

In step 204, a user input of a change to at least one of the degrees of freedom is received. The user input may be received via the GUI. The input by the user may be a change to at least one of: translation of the moving jaw portion 100 relative to the fixed jaw portion 104 in the X direction, translation of the moving jaw portion 100 relative to the fixed jaw portion 104 in the Y direction, rotation of the moving jaw portion 100 relative to the fixed jaw portion 104 about the Z axis, and the position of the control point 110, e.g., along the Y axis.

In step 206, in response to the user input, at least one of the other degrees of freedom is automatically adjusted to minimize a vertical distance between the fixed jaw portion 104 and moving jaw portion 100, possibly in their modified form to reflect a maximum burring depth, to determine a virtual occlusion 108. The vertical distance may be the distance in the Z direction from the control point 110 to the origin of the WCS. The automatic adjustment may be constrained to so that distances between opposing surfaces of the moving jaw portion 100 and fixed jaw portion 104 are positive. The degree of freedom that is automatically adjusted may be at least one of: translation of the moving jaw portion 100 relative to the fixed jaw portion 104 in the Z direction, rotation of the moving jaw portion 100 relative to the fixed jaw portion 104 about the X axis, and rotation of the moving jaw portion 100 relative to the fixed jaw portion 104 about the Y axis.

In a different embodiment, in step 204, user input of a change to at least one of the degrees of freedom is received. The user input may be received via the GUI. The input by the user may be a change to at least one of: translation of the moving jaw portion 100 relative to the fixed jaw portion 104 in the X direction, translation of the moving jaw portion 100 relative to the fixed jaw portion 104 in the Y direction, rotation of the moving jaw portion 100 relative to the fixed jaw portion 104 about the X axis, rotation of the moving jaw portion 100 relative to the fixed jaw portion 104 about the Z axis, and the position of the control point 110, e.g., along the X axis.

In such different embodiments, in step 206, in response to the user input, at least one of the other degrees of freedom is automatically adjusted to minimize a vertical distance between the fixed jaw portion 104 and moving jaw portion 100, possibly in their modified form to reflect a maximum burring depth, to determine a virtual occlusion 108. The vertical distance may be the distance in the Z direction from the control point 110 to the origin. The automatic adjustment may be constrained to so that distances between opposing surfaces of the moving jaw portion 100 and fixed jaw portion 104 are positive. The degree of freedom that is automatically adjusted may be at least one of: translation of the moving jaw portion 100 relative to the fixed jaw portion 104 in the Z direction, and rotation of the moving jaw portion 100 relative to the fixed jaw portion 104 about the Y axis.

In step 208, the moving jaw portion 100 and the fixed jaw portion 104 are represented in the determined occlusion 108. This may, e.g., comprise displaying original and/or modified 3-D representations of the jaw portions in the determined occlusion 108, and/or displaying one or more occlusionograms. The one or more occlusionograms may be based on distance measurements between original 3-D representations, modified 3-D representations or a combination of both. The above steps may be iteratively performed so as to represent a new determined occlusion 108 in response to each change input by the user.

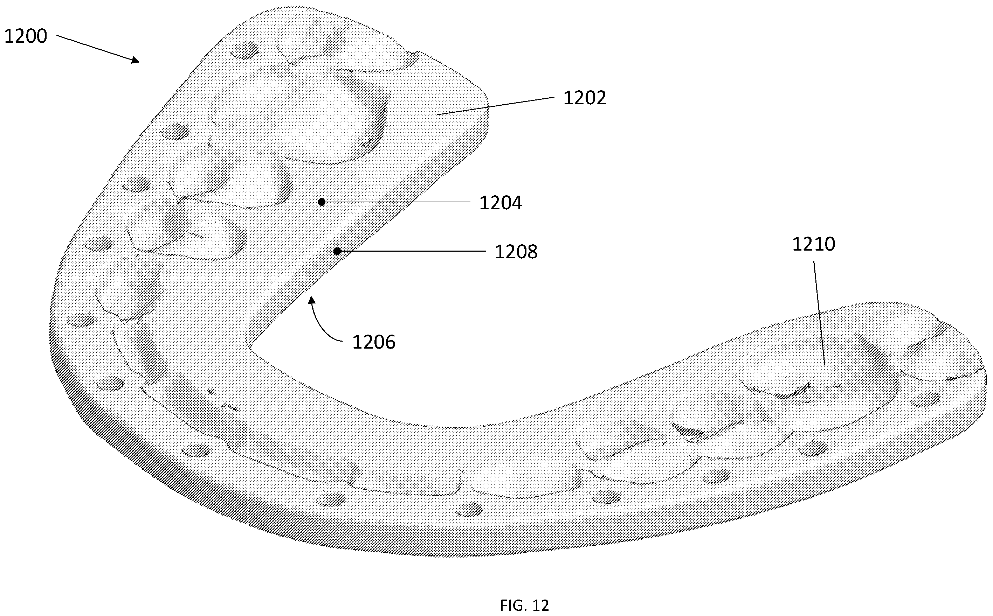

According to certain embodiments, a surgical guide may be formed based on the determined occlusion. For example, the surgical guide may be 3-D printed. The surgical guide may be configured to guide the patient's teeth into a position corresponding to the determined occlusion during orthognathic surgery. For example, the surgical guide may take the form of a splint. For example, as is illustrated in Figure 12, a 3-D shape 1200 may be determined for the guide. The 3-D shape 1200 may be formed by first creating a substantially disk-shaped or wedge-shaped body 1202, having a top surface 1204, a bottom surface 1206, opposing the top surface, and an outer edge 1208. The outer edge may, for example, roughly mimic an outer offset of the contour of the larger dentition of the two jaws, viewed from the top. The body may, for example, have a thickness of between 1mm and 5mm. Next, indentations 1210 may be formed in the top and bottom surfaces that are complementary to portions of the 3-D shapes of the jaw portions in the determined occlusion. For example, the body may be positioned centered around the occlusion plane and a Boolean subtraction may be performed in which the 3-D model(s) of the jaw portions are subtracted from the body. The surgical guide may be manufactured based on this 3-D shape, e.g., using any suitable additive-manufacturing technology, such as stereo lithography, selective laser sintering or selective laser melting, in any suitable material, such as a biocompatible polymer or metal.

According to certain embodiments, the unique way in which the components interact provides for improved determination of the virtual occlusion 108. Specifically, the "distribution of work" between the user and the algorithm, which is achieved by dividing the six DOFs into three or four under user control and three or two, respectively, under control of the algorithm, is unique. The DOFs that the algorithm controls are variables that strongly affect whether or not the teeth make contact: a small alteration will tend to move the collision points vertically. On the other hand, an alteration to the DOFs under user control will tend to move collision points horizontally, causing the teeth to make contact at different points.

Due to this division of the six DOFs, the system provides a "tactile" user experience, approximating the act of physically pushing two dental casts together. The user only has to do part of the positioning while the algorithm "pushes" the teeth into three-point contact. The role of the control point 110 in the algorithm also contributes to this improved user experience: when the action of the algorithm is interpreted as pushing the jaws together, the control point 110 is the point where the force is being applied.

In embodiments that allow the user to manipulate four DOFs to obtain two-point contact rather than three DOFs to obtain three-point contact, there is a different trade-off between the number of parameters the user must control (and therefore the burden placed on the user) and the degree of control that the user has over the resulting occlusion, with the balance shifted towards a greater degree of control. This functionality may be optionally provided to the user, to be used primarily when the shape of the patient's jaws or the clinical conditions make this increased degree of control necessary.

In addition, according to some embodiments the specific way in which the optimization algorithm is used provides unique benefits. In certain embodiments, the search space of the algorithm consists of only two or three variables, which limits the dimensionality of the problem, making it easier to solve numerically. Using the control point's vertical displacement as the goal function makes the latter fast to evaluate, as it can be expressed as a simple function of the optimization variables. This helps reduce the algorithm's running time, which in turn makes the system feel more responsive. Thus, the process of defining a virtual occlusion is accelerated and allows for a smooth interaction among people involved in the surgical planning process, e.g., between the engineer operating the systems disclosed herein and the surgeon.

Embodiments of the techniques discuss herein can make the process to define a virtual occlusion much faster, as the user does not have to spend any time bringing the teeth into contact. In addition, the user only needs to manipulate a limited set of parameters (the three or four DOFs under user control and the control point), which simplifies the process, reduces training need and improves reproducibility. The three or four available parameters may have a relevant clinical interpretation, which makes their manipulation straightforward. Furthermore, this may allow for a more natural discussion among people involved in the surgical planning process, e.g., between engineer and surgeon during online planning sessions for orthognathic surgery. In addition, the greater speed and ease of use can allow for more alternative occlusions to be compared for the same case, potentially improving the quality of the treatment plan.

The specific definition of coordinate systems and Euler angles discussed with respect to certain embodiments is only an example, and it should be understood that the definition can be changed, e.g., by renaming coordinate axes, flipping them or even rotating them, and still be used with the embodiments herein. Furthermore, a different formalism could be used for the embodiments discussed herein to represent the 3D rotation between LCS and WCS, such as a rotation vector. Each of the different embodiments may provide that the pose of the moving jaw portion is represented by six DOFs (three for rotation and three for translation), and three of these (two for rotation and one for translation) or two of these (one for rotation and one for translation) are manipulated by the algorithm to bring the teeth into contact, with the remaining DOFs being under direct user control.

The collision points and collision triangles used to represent contact between the teeth in certain embodiments discussed herein could be changed in several ways. For example, collision points could be defined on the fixed jaw portion rather than on the moving jaw portion, or even on both. Similarly, in certain embodiments, collision triangles could be defined on the fixed jaw portion rather than on the moving jaw portion, or even on both. In certain embodiments, different methods could be used to express the distance between both jaws, such as not using points and triangles. For example, in certain embodiments distance between jaw portions can be expressed using signed distance fields defined on implicit surfaces. Each of the different embodiments may provide that a series of distances between both jaw portions is calculated, distributed over the surface of the teeth of one (or both) jaws. These distances may be positive when the teeth are not in contact, zero when the teeth come into contact and negative when the teeth's surfaces intersect (the 3D objects overlap). Alternatively, the signs could be reversed (positive for overlap, negative for no contact).

In certain embodiments, the optimization algorithm constrains all distance values to be positive (greater than 0). Alternatively, in certain embodiments, the optimization algorithm may constrain the distances to be greater than a (small) negative number, such as a number between - 1mm and 0mm, more particularly -0.1mm, -0.2mm, -0.3mm, -0.4mm or -0.5mm. In addition, in certain embodiments, this number could vary over the surface of the teeth. This may allow for a small amount of overlap, which can be useful to make sure the teeth are in contact even despite small precision errors, or to simulate that part of a tooth is removed, for example by burring.

As another alternative, in some embodiments, the optimization constraints can be replaced with one or more penalty terms added to the goal function. The value of these penalty terms could be zero or very low if there is no contact but rise rapidly with increasing interpenetration of both surfaces. The signed distance between a point of one of the jaw portions and the surface of the other jaw portion could be used for this. In certain embodiments, the penalty term would then be zero for positive signed distances (point outside of the surface) and equal to the absolute value or square of negative signed distances (point inside of the surface, i.e., contact).

In certain embodiments, the optimization goal function can be replaced with anything that expresses the "closeness" of both sets of teeth with a single number. An example could be the sum of distances or squared distances between points defined on either jaw portion. In addition, in certain embodiments, alternatives are possible for the control point used to let the user control where both jaw portions will make contact. In certain embodiments, the user could be allowed to define one or more regions on the surface of the teeth in which one or more contact points must be located, or where no contact points may be located. This could be useful in cases where for clinical reasons no contact is desired at (for example) the incisor teeth, or in cases where contact should be avoided at some of the teeth. The regions on the surface of the teeth in which one or more contact points must be located, or where no contact points may be located, could be indicated by the user using a paintbrush-like tool to mark sections of the surface of the teeth. Many other ways of interactively indicating a region are possible. For example, the user may use a lasso tool or a polygon lasso tool. Alternatively, the user may specify a line in the LCS or WCS X-Y plane. The sections of the surface of the teeth anterior or posterior to a vertical plane (parallel to the Z axis) through this line could be used as the aforementioned regions.

In certain embodiments, a GUI or interface used for the system may be different than the example GUI discussed herein without affecting the functionality of the system. For example, other interfaces can provide at least two functions: (1) allowing the user to select the parameters under user control, e.g., three or four DOFs and the control point; and (2) presenting the resulting occlusion to the user. Any interface that performs both functions can be used in conjunction with the techniques discussed herein. For example, the buttons provided as an example could be replaced with e.g., sliders and the occlusionograms could be replaced or supplemented by other methods of visualizing the distances between teeth, e.g., by showing a series of cross-sections or by overlaying a colormap on the 3-D model. For convenience, the GUI of any embodiment disclosed herein may offer the possibility to selectively hide or show one or more jaw portions in the 3-D model. Other alternative input methods to replace the buttons on the GUI include the use of one or more input devices that are conventionally used to provide user inputs to a computer, for example computer mice, joysticks, trackpads, touch screens, trackballs, motion controllers, hand or eye tracking devices, or 6-degree-of-freedom input devices such as the 3Dconnexion SpaceMouse (3Dconnexion GmbH, München, Germany). When using one or more of such input devices, the input values for one or more DOFs that are under control of the user in the disclosed method may be directly derived from corresponding axes or degrees of freedom provided by the input devices. For example, the X and Y translation could be directly controlled by the left/right and forward/backward movements of a computer mouse, so that when the user moves the mouse left or right, the moving jaw portion moves in the X direction and when the user moves the mouse forward or backward, the moving jaw portion moves in the Y direction. With this set-up, the moving jaw portion's translation in the X-Y plane will mimic the movement of the user's mouse. Simultaneously, the rotation around the Z axis may be controlled by e.g., the scroll wheel, or even by a different input device.

In certain embodiments, the system may utilize an augmented reality (AR) or virtual reality (VR) system, devices, or parts thereof, for display and/or user interaction, such as providing the interface. For example, instead of using buttons or sliders, the user would be able to use an input method provided by the AR or VR system to manipulate the parameters under user control. In certain embodiments, this input method includes some form of motion capture performed by the AR/VR system, e.g., hand gestures or a motion controller. In certain embodiments, voice commands may be used. In certain embodiments, the combination of an AR/VR-based user interface with the embodiments discussed herein limiting user manipulation of the moving jaw portion to three or four DOFs can be advantageous. For example, controlling six degrees of freedom simultaneously to manually set an occlusion would be difficult even with motion controls, but the use of only three DOFs reduces the number of parameters, e.g., to four (three DOFs and a control point). In certain embodiments, the resulting occlusion would be presented to the user as repositioned 3D models, potentially with occlusionograms superimposed on them. Further, the occlusionograms may also be superimposed on the patient. This may be similar to the information displayed on an ordinary computer screen, but the AR/VR interface would make it easier to view the 3D models from different angles. In certain embodiments, if the AR/VR system uses hand tracking for user input, the interface can closely mimic the traditional method of physically positioning dental casts in occlusion by positioning the 3D models (physical and/or virtual) so they follow the user's hands. In addition, in certain embodiments, if an AR system is able to track the patient's upper or lower jaw portions, the 3D models can be shown superimposed on the patient's face in real time. This would allow the user to easily judge the repositioning of the moving jaw in relation to the shape of the patient's face.

According to embodiments, the GUI may represent an orthognathic surgical procedure. For example, in the GUI, the represented maxilla portion may be cut and repositioned in an initial position prior to determining the virtual occlusion 108. Alternatively, the GUI may represent an iterative process where the positions of the split maxilla pieces are repositioned based on the virtual occlusion 108 that is determined after each user input of one or more of the three or four DOFs under the user's control.

In certain embodiments, the GUI may represent an orthognathic surgical procedure in which individual parts of a split maxilla and/or a split mandible are repositioned. The GUI may allow repositioning all maxilla or all mandible parts as a whole or repositioning a single mandible or maxilla part, where the user selects which of both jaws is to be repositioned and whether the entire jaw (consisting of all portions) or a single part of the selected jaw is to be repositioned. Based on the user's selection, either a single part of a jaw will be treated as the moving jaw portion, or all parts of the selected jaw will be merged into one object and the result treated as the moving jaw portion. This can be an iterative process, where the user can alternate between repositioning individual parts of the selected jaw and repositioning the entire jaw as a whole. When transitioning from repositioning an individual part to repositioning the entire jaw, the parts of the selected jaw can be merged in such a way that their relative position and orientation resulting from the repositioning of individual parts is maintained. This can be achieved by applying a transformation to their 3D geometry, e.g., a 3-D model, (which includes collision points and collision triangles) so all parts are defined in the LCS of the entire jaw. When transitioning from repositioning the entire jaw to repositioning an individual part, this operation can be reversed by expressing all parts in their individual local coordinate systems while setting the location of the LCS origins and the orientation of the LCS axes in such a way that the parts' positions and orientations in the WCS remain the same as they were when merged into a single part representing the entire jaw. Furthermore, the GUI may allow the user to alternate between manipulating different parts of a split mandible or a split maxilla. While the user is manipulating one part, based on a preference selected by the user, the GUI may or may not visualize the other jaw parts that the user is not manipulating.

Though certain embodiments are described with respect to orthognathics, the techniques discussed herein are similarly applicable for planning orthodontic treatment based on intra-oral scans (including plaster casts) or other suitable procedures, such as in craniomaxillofacial procedures (e.g. in trauma, reconstruction surgeries, etc.), surgical procedures wherein guides or implants are placed, or non-medical applications wherein two parts of one or more objects need to be aligned with respect to one another for a stable position, etc.

For example, techniques described herein may be used in the design of surgical guides (e.g., 3D printed drill guides). In particular, they could be used to help identify alternative poses in which a guide could make a stable contact with the bone in a different position than intended for the surgical plan.

Certain embodiments discussed herein may be implemented as a computer program running on a computer. In certain aspects, input data to the program includes 3-D models of (at least) the teeth of both jaw portions 100, 104.

To implement the program, in certain embodiments, a GUI framework is used to present the occlusion to the user and to allow the user to determine values for the parameters that are under user control. Furthermore, the computer program may include instructions for determining the LCS 102 from these parameters and performing transformations from LCS 102 to WCS 106. The computer program may include instructions for calculating the distance to the closest point on the collision triangles 114 for each collision point 112. Finally, an optimization algorithm may be used.

The systems and methods described herein can be operated and performed by, for example, computing devices, such as desktop computers, portable computers, portable electronic devices, tablet computers, smart phones, and other computerized devices. In some implementations, the methods described herein may be performed by native software applications while in others they may be performed in server-client implementations. For example, in some implementations, software configured to perform the methods described herein may be hosted by a remote server or a cloud-based system. In some cases, various aspects of the systems and methods described herein may be distributed across different computing devices. For example, the method steps described herein related to presenting a GUI, receiving user input and determining virtual occlusion do not require great computation power and are therefore well suited to be performed by mobile devices, such as portable computers, portable electronic devices, tablet computers or smart phones, while other steps, e.g., relating to the acquisition of medical image data, the conversion of medical image data into 3-D representations of the fixed and moving jaw portions, or the acquisition of 3-D scans of the fixed and moving jaw portions may be performed by more powerful computing devices, such as desktop computers or workstations, or dedicated devices equipped with image acquisition or scanning functionality.

Further, the systems and methods described herein can be operated and performed by, for example a medical professional, such as a surgeon, doctor, or nurse, or by a non-medical professional, such as a clinical technician, design engineer, implant manufacturer, or a patient (e.g., who is walked through the surgery before the actual surgery).

The preceding description is provided to enable any person skilled in the art to practice the various embodiments described herein. The examples discussed herein are not limiting of the scope, applicability, or embodiments set forth in the claims. Various modifications to these embodiments will be readily apparent to those skilled in the art, and the generic principles defined herein may be applied to other embodiments. For example, changes may be made in the function and arrangement of elements discussed without departing from the scope of the disclosure. Various examples may omit, substitute, or add various procedures or components as appropriate. For instance, the methods described may be performed in an order different from that described, and various steps may be added, omitted, or combined. Also, features described with respect to some examples may be combined in some other examples. For example, an apparatus may be implemented, or a method may be practiced using any number of the aspects set forth herein. In addition, the scope of the disclosure is intended to cover such an apparatus or method that is practiced using other structure, functionality, or structure and functionality in addition to, or other than, the various aspects of the disclosure set forth herein. It should be understood that any aspect of the disclosure disclosed herein may be embodied by one or more elements of a claim.

As used herein, the word "exemplary" means "serving as an example, instance, or illustration." Any aspect described herein as "exemplary" is not necessarily to be construed as preferred or advantageous over other aspects.

As used herein, the words "fixed" and "stationary" may be used interchangeably.

As used herein, a phrase referring to "at least one of" a list of items refers to any combination of those items, including single members. As an example, "at least one of: a, b, or c" is intended to cover a, b, c, a-b, a-c, b-c, and a-b-c, as well as any combination with multiples of the same element (e.g., a-a, a-a-a, a-a-b, a-a-c, a-b-b, a-c-c, b-b, b-b-b, b-b-c, c-c, and c-c-c or any other ordering of a, b, and c).

As used herein, the term "determining" encompasses a wide variety of actions. For example, "determining" may include calculating, computing, processing, deriving, investigating, looking up (e.g., looking up in a table, a database or another data structure), ascertaining and the like. Also, "determining" may include receiving (e.g., receiving information), accessing (e.g., accessing data in a memory) and the like. Also, "determining" may include resolving, selecting, choosing, establishing and the like.

The methods disclosed herein comprise one or more steps or actions for achieving the methods. The method steps and/or actions may be interchanged with one another without departing from the scope of the claims. In other words, unless a specific order of steps or actions is specified, the order and/or use of specific steps and/or actions may be modified without departing from the scope of the claims. Further, the various operations of methods described above may be performed by any suitable means capable of performing the corresponding functions. The means may include various hardware and/or software component(s) and/or module(s), including, but not limited to a circuit, an application specific integrated circuit (ASIC), or processor. Generally, where there are operations illustrated in figures, those operations may have corresponding counterpart means-plus-function components with similar numbering.

The various illustrative logical blocks, modules and circuits described in connection with the present disclosure may be implemented or performed with a general-purpose processor, a digital signal processor (DSP), an application specific integrated circuit (ASIC), a field programmable gate array (FPGA) or other programmable logic device (PLD), discrete gate or transistor logic, discrete hardware components, or any combination thereof designed to perform the functions described herein. A general-purpose processor may be a microprocessor, but in the alternative, the processor may be any commercially available processor, controller, microcontroller, or state machine. A processor may also be implemented as a combination of computing devices, e.g., a combination of a DSP and a microprocessor, a plurality of microprocessors, one or more microprocessors in conjunction with a DSP core, or any other such configuration.

A processing system may be implemented with a bus architecture. The bus may include any number of interconnecting buses and bridges depending on the specific application of the processing system and the overall design constraints. The bus may link together various circuits including a processor, machine-readable media, and input/output devices, among others. A user interface (e.g., keypad, display, mouse, joystick, etc.) may also be connected to the bus. The bus may also link various other circuits such as timing sources, peripherals, voltage regulators, power management circuits, and other circuit elements that are well known in the art, and therefore, will not be described any further. The processor may be implemented with one or more general-purpose and/or special-purpose processors. Examples include microprocessors, microcontrollers, DSP processors, and other circuitry that can execute software. Those skilled in the art will recognize how best to implement the described functionality for the processing system depending on the particular application and the overall design constraints imposed on the overall system.

If implemented in software, the functions may be stored or transmitted over as one or more instructions or code on a computer-readable medium. Software shall be construed broadly to mean instructions, data, or any combination thereof, whether referred to as software, firmware, middleware, microcode, hardware description language, or otherwise. Computer-readable media include both computer storage media and communication media, such as any medium that facilitates transfer of a computer program from one place to another. The processor may be responsible for managing the bus and general processing, including the execution of software modules stored on the computer-readable storage media. A computer-readable storage medium may be coupled to a processor such that the processor can read information from, and write information to, the storage medium. In the alternative, the storage medium may be integral to the processor. By way of example, the computer-readable media may include a transmission line, a carrier wave modulated by data, and/or a computer readable storage medium with instructions stored thereon separate from the wireless node, all of which may be accessed by the processor through the bus interface. Alternatively, or in addition, the computer-readable media, or any portion thereof, may be integrated into the processor, such as the case may be with cache and/or general register files. Examples of machine-readable storage media may include, by way of example, RAM (Random Access Memory), flash memory, ROM (Read Only Memory), PROM (Programmable Read-Only Memory), EPROM (Erasable Programmable Read-Only Memory), EEPROM (Electrically Erasable Programmable Read-Only Memory), registers, magnetic disks, optical disks, hard drives, or any other suitable storage medium, or any combination thereof. The machine-readable media may be embodied in a computer-program product.

A software module may comprise a single instruction, or many instructions, and may be distributed over several different code segments, among different programs, and across multiple storage media. The computer-readable media may comprise a number of software modules. The software modules include instructions that, when executed by an apparatus such as a processor, cause the processing system to perform various functions. The software modules may include a transmission module and a receiving module. Each software module may reside in a single storage device or be distributed across multiple storage devices. By way of example, a software module may be loaded into RAM from a hard drive when a triggering event occurs. During execution of the software module, the processor may load some of the instructions into cache to increase access speed. One or more cache lines may then be loaded into a general register file for execution by the processor. When referring to the functionality of a software module, it will be understood that such functionality is implemented by the processor when executing instructions from that software module.

The following claims are not intended to be limited to the embodiments shown herein but are to be accorded the full scope consistent with the language of the claims. Within a claim, reference to an element in the singular is not intended to mean "one and only one" unless specifically so stated, but rather "one or more." Unless specifically stated otherwise, the term "some" refers to one or more.

## Claims

1. A computer-implemented method of determining a virtual occlusion, comprising:
obtaining (200) a 3-D representation of a patient's first jaw portion and the patient's second jaw portion, the second jaw portion being opposite to and moveable with respect to the first jaw portion;
with the 3-D representation, setting and representing (202) in a graphic user interface (GUI) an initial position of the second jaw portion relative to the first jaw portion, the initial position being defined by a control point on the second jaw portion and the second jaw portion having six pre-determined degrees of freedom relative to a coordinate system with an origin fixed relative to the first jaw portion;
receiving (204) user input of changes to at least one of the degrees of freedom, wherein the user input of changes comprises a change to a position of the control point relative to the second jaw portion;
automatically adjusting (206) at least one of the other degrees of freedom to minimize a vertical distance of the control point to the origin while constraining distances between opposed surfaces of the first and second jaw portions to be positive, thereby determining an occlusion between the first and second jaw portions; and
representing (208), in the GUI, the first and second jaw portions in the determined occlusion.

2. The method of claim 1, wherein the user input of changes further comprises a change to at least one of: a position of the second jaw portion in a direction in the coordinate system corresponding to the patient's left-right direction, the position of the second jaw portion in a direction in the coordinate system corresponding to the patient's anterior-posterior direction, or a rotation of the second jaw portion around a direction in the coordinate system corresponding to the patient's upward-downward direction; and
wherein automatically adjusting at least one of the other degrees of freedom comprises automatically adjusting at least one of: a position of the second jaw portion in a direction in the coordinate system corresponding to the patient's upward-downward direction, a rotation of the second jaw portion around a direction in the coordinate system corresponding to the patient's left-right direction, or a rotation of the second jaw portion around a direction in the coordinate system corresponding to the patient's anterior-posterior direction.

3. The method of claim 1, wherein the user input of changes further comprises a change to at least one of: a position of the second jaw portion in a direction in the coordinate system corresponding to the patient's left-right direction, the position of the second jaw portion in a direction in the coordinate system corresponding to the patient's anterior-posterior direction, a rotation of the second jaw portion around a direction in the coordinate system corresponding to the patient's upward-downward direction, or a rotation of the second jaw portion around a direction in the coordinate system corresponding to the patient's left-right direction; and
wherein automatically adjusting at least one of the other degrees of freedom comprises automatically adjusting at least one of: a position of the second jaw portion in a direction in the coordinate system corresponding to the patient's upward- downward direction, or a rotation of the second jaw portion around a direction in the coordinate system corresponding to the patient's anterior-posterior direction.

4. The method of any one of claims 1-3, wherein the user input of change to the position of the control point relative to the second jaw portion comprises change to the position of the control point in the direction in the coordinate system corresponding to the patient's anterior-posterior direction.

5. The method of claim 1, wherein the user input of changes further comprises a change to at least one of: a position of the second jaw portion in a direction in the coordinate system corresponding to the patient's mesial-distal direction, the position of the second jaw portion in a direction in the coordinate system corresponding to the patient's buccal-lingual direction, a rotation of the second jaw portion around a direction in the coordinate system corresponding to the patient's upward-downward direction, or a rotation of the second jaw portion around a direction in the coordinate system corresponding to the patient's mesial-distal direction; and
wherein automatically adjusting at least one of the other degrees of freedom comprises automatically adjusting at least one of: a position of the second jaw portion in a direction in the coordinate system corresponding to the patient's upward- downward direction, or a rotation of the second jaw portion around a direction in the coordinate system corresponding to the patient's buccal-lingual direction.

6. The method of any one of claims 1-5, wherein:
the first jaw portion comprises at least part of the patient's maxilla, and
the second jaw portion comprises at least part of the patient's mandible.

7. The method of any one of claims 1-5, wherein:
the first jaw portion comprises at least part of the patient's mandible, and
the second jaw portion comprises at least part of the patient's maxilla.

8. The method of any one of claims 1-7, wherein the GUI comprises a 3-D model of each of the first jaw portion and the second jaw portion.

9. The method of claim 8, further comprising displaying one or more of:
a distance between the first and second jaw portions in the determined occlusion as a color map on the 3-D model;
contact points between the first and second jaw portions in the determined occlusion on the 3-D model;
approximate contact points between the first and second jaw portions in the determined occlusion on the 3-D model; and
a convex hull around contact points and/or approximate contact points between the first and second jaw portions in the determined occlusion on the 3-D model.

10. The method of any one of claims 1-9, wherein the GUI further comprises an occlusionogram of at least one of the first jaw portion and the second jaw portion.

11. The method of claim 10, further comprising displaying one or more of:
contact points between the first and second jaw portions in the determined occlusion on the occlusionogram;
approximate contact points between the first and second jaw portions in the determined occlusion on the occlusionogram; and
a convex hull around contact points and/or approximate contact points between the first and second jaw portions in the determined occlusion on the occlusionogram.

12. The method of any one of claims 1-11, further comprising updating the representation of the first and second jaw portions in the GUI in the occlusion determined in response to additional user input of changes to at least one of the degrees of freedom and corresponding automatic adjustments of at least one of the other degrees of freedom.

13. The method of any one of claims 1-12, further comprising:
receiving user input indicating a region of at least one of the first and second jaw portions and assigning a maximum burring depth to said region; and
generating at least one modified 3-D model representing the at least one of the first and second jaw portions based on the indicated region and the maximum burring depth,
wherein automatically adjusting at least one of the other degrees of freedom comprises constraining distances between opposed surfaces of the first and second jaw portions to be positive based on the at least one modified 3-D model.

14. The method of claim 13, wherein the GUI comprises an occlusionogram of at least one of the first jaw portion and the second jaw portion based on the at least one modified 3-D model.

15. A computer-implemented method of forming a surgical guide based on a determined virtual occlusion, comprising:
determining a virtual occlusion between a patient's first jaw portion and the patient's second jaw portion using the method of any one of claims 1-14; and
forming a surgical guide to guide the patient's teeth into the determined occlusion during orthognathic surgery.

16. The method of claim 15, wherein forming the surgical guide comprises manufacturing the surgical guide by means of an additive manufacturing technology.

17. The method of claim 15 or 16, wherein the surgical guide comprises a guide body, said guide body comprising a top surface, a bottom surface opposing the top surface and an edge, said top and bottom surfaces comprising indentations shaped complementary to surface portions of the patient's first and second jaw portions in the determined occlusion.

18. A system for determining a virtual occlusion, comprising:
a graphic user interface (GUI); and
a processor configured to execute the method of any one of claims 1-17.

19. A non-transitory computer-readable storage medium, having instructions stored therein, which, when executed by a computing device, causes the computing device to carry out the method according to any one of claims 1-17.

## Patentansprüche

1. Computergestütztes Verfahren zur Bestimmung einer virtuellen Okklusion, umfassend:
Erhalten (200) einer 3D-Darstellung eines ersten Kieferabschnitts eines Patienten und eines zweiten Kieferabschnitts des Patienten, wobei der zweite Kieferabschnitt dem ersten Kieferabschnitt gegenüberliegt und relativ zu diesem beweglich ist;
mit der 3D-Darstellung Einstellen und Darstellen (202) einer Ausgangsposition des zweiten Kieferabschnitts relativ zum ersten Kieferabschnitt in einer grafischen Benutzeroberfläche (GUI), wobei die Ausgangsposition durch einen Kontrollpunkt auf dem zweiten Kieferabschnitt definiert ist und der zweite Kieferabschnitt sechs vorbestimmte Freiheitsgrade relativ zu einem Koordinatensystem mit einem relativ zum ersten Kieferabschnitt festen Ursprung aufweist;
Empfangen (204) einer Benutzereingabe von Änderungen an mindestens einem der Freiheitsgrade, wobei die Benutzereingabe von Änderungen eine Änderung an einer Position des Kontrollpunkts relativ zum zweiten Kieferabschnitt umfasst;
automatisches Anpassen (206) mindestens eines der anderen Freiheitsgrade, um einen vertikalen Abstand des Kontrollpunkts zum Ursprung zu minimieren, während die Abstände zwischen gegenüberliegenden Oberflächen des ersten und des zweiten Kieferabschnitts positiv gehalten werden, wodurch eine Okklusion zwischen dem ersten und dem zweiten Kieferabschnitt bestimmt wird; und
Darstellen (208) des ersten und des zweiten Kieferabschnitts in der bestimmten Okklusion in der GUI.

2. Verfahren nach Anspruch 1, wobei die Benutzereingabe von Änderungen ferner eine Änderung an mindestens einem der folgenden Elemente umfasst: eine Position des zweiten Kieferabschnitts in einer Richtung im Koordinatensystem, die der Links-Rechts-Richtung des Patienten entspricht, die Position des zweiten Kieferabschnitts in einer Richtung im Koordinatensystem, die der anterior-posterior-Richtung des Patienten entspricht, oder eine Drehung des zweiten Kieferabschnitts um eine Richtung im Koordinatensystem, die der Aufwärts-Abwärts-Richtung des Patienten entspricht; und
wobei das automatische Anpassen mindestens eines der anderen Freiheitsgrade das automatische automatische Anpassen mindestens eines der folgenden Elemente umfasst: eine Position des zweiten Kieferabschnitts in einer Richtung im Koordinatensystem, die der Aufwärts-Abwärts-Richtung des Patienten entspricht, eine Drehung des zweiten Kieferabschnitts um eine Richtung im Koordinatensystem, die der Links-Rechts-Richtung des Patienten entspricht, oder eine Drehung des zweiten Kieferabschnitts um eine Richtung im Koordinatensystem, die der anterior-posterior-Richtung des Patienten entspricht.

3. Verfahren nach Anspruch 1, wobei wobei die Benutzereingabe von Änderungen ferner eine Änderung an mindestens einem der folgenden Elemente umfasst: eine Position des zweiten Kieferabschnitts in einer Richtung im Koordinatensystem, die der Links-Rechts-Richtung des Patienten entspricht, die Position des zweiten Kieferabschnitts in einer Richtung im Koordinatensystem, die der anterior-posterior-Richtung des Patienten entspricht, eine Drehung des zweiten Kieferabschnitts um eine Richtung im Koordinatensystem, die der Aufwärts-Abwärts-Richtung des Patienten entspricht, oder eine Drehung des zweiten Kieferabschnitts um eine Richtung im Koordinatensystem, die der Links-Rechts-Richtung des Patienten entspricht; und
wobei das automatische Anpassen mindestens eines der anderen Freiheitsgrade das automatische Anpassen mindestens eines der folgenden Elemente umfasst: eine Position des zweiten Kieferabschnitts in einer Richtung im Koordinatensystem, die der Aufwärts-Abwärts-Richtung des Patienten entspricht, oder eine Drehung des zweiten Kieferabschnitts um eine Richtung im Koordinatensystem, die der anterior-posterior-Richtung des Patienten entspricht.

4. Verfahren nach einem der Ansprüche 1 - 3, wobei die Benutzereingabe einer Änderung an der Position des Kontrollpunkts relativ zum zweiten Kieferabschnitt eine Änderung an der Position des Kontrollpunkts in der Richtung im Koordinatensystem umfasst, die der anterior-posterior-Richtung des Patienten entspricht.

5. Verfahren nach Anspruch 1, wobei die Benutzereingabe von Änderungen ferner eine Änderung an mindestens einem der folgenden Elemente umfasst: eine Position des zweiten Kieferabschnitts in einer Richtung im Koordinatensystem, die der mesial-distal-Richtung des Patienten entspricht, die Position des zweiten Kieferabschnitt in einer Richtung im Koordinatensystem, die der bukkal-lingual-Richtung des Patienten entspricht, eine Drehung des zweiten Kieferabschnitts um eine Richtung im Koordinatensystem, die der Aufwärts-Abwärts-Richtung des Patienten entspricht, oder eine Drehung des zweiten Kieferabschnitts um eine Richtung im Koordinatensystem, die der mesial-distal-Richtung des Patienten entspricht; und
wobei das automatische Anpassen mindestens eines der anderen Freiheitsgrade das automatische Anpassen mindestens eines der folgenden Elemente umfasst: eine Position des zweiten Kieferabschnitts in einer Richtung im Koordinatensystem, die der Aufwärts-Abwärts-Richtung des Patienten entspricht, oder eine Drehung des zweiten Kieferabschnitts um eine Richtung im Koordinatensystem, die der bukkal-lingual-Richtung des Patienten entspricht.

6. Verfahren nach einem der Ansprüche 1 - 5, wobei:
der erste Kieferabschnitt mindestens einen Teil des Oberkiefers des Patienten umfasst und
der zweite Kieferabschnitt mindestens einen Teil des Unterkiefers des Patienten umfasst.

7. Verfahren nach einem der Ansprüche 1 - 5, wobei:
der erste Kieferabschnitt mindestens einen Teil des Unterkiefers des Patienten umfasst und
der zweite Kieferabschnitt mindestens einen Teil des Oberkiefers des Patienten umfasst.

8. Verfahren nach einem der Ansprüche 1 - 7, wobei die GUI ein 3D-Modell sowohl des ersten Kieferabschnitts als auch des zweiten Kieferabschnitts umfasst.

9. Verfahren nach Anspruch 8, ferner umfassend das Anzeigen von einem oder mehreren der folgenden Elemente:
ein Abstand zwischen dem ersten und dem zweiten Kieferabschnitt in der bestimmten Okklusion als Farbkarte auf dem 3D-Modell;
Kontaktpunkte zwischen dem ersten und dem zweiten Kieferabschnitt in der bestimmten Okklusion auf dem 3D-Modell;
ungefähre Kontaktpunkte zwischen dem ersten und dem zweiten Kieferabschnitt in der bestimmten Okklusion auf dem 3D-Modell; und
eine konvexe Hülle um Kontaktpunkte und/oder ungefähre Kontaktpunkte zwischen dem ersten und dem zweiten Kieferabschnitt in der bestimmten Okklusion auf dem 3D-Modell.

10. Verfahren nach einem der Ansprüche 1 - 9, wobei die GUI ferner ein Okklusogramm mindestens eines der ersten und zweiten Kieferabschnitte umfasst.

11. Verfahren nach Anspruch 10, ferner umfassend das Anzeigen von einem oder mehreren der folgenden Elemente:
Kontaktpunkte zwischen dem ersten und dem zweiten Kieferabschnitt in der bestimmten Okklusion auf dem Okklusogramm;
ungefähre Kontaktpunkten zwischen dem ersten und dem zweiten Kieferabschnitt in der bestimmten Okklusion auf dem Okklusogramm; und
eine konvexe Hülle um Kontaktpunkte und/oder ungefähre Kontaktpunkte zwischen dem ersten und dem zweiten Kieferabschnitt in der bestimmten Okklusion auf dem Okklusogramm.

12. Verfahren nach einem der Ansprüche 1 - 11, ferner umfassend das Aktualisieren der Darstellung des ersten und des zweiten Kieferabschnitts in der GUI in der bestimmten Okklusion als Reaktion auf zusätzliche Benutzereingaben von Änderungen an mindestens einem der Freiheitsgrade und entsprechende automatische Anpassungen von mindestens einem der anderen Freiheitsgrade.

13. Verfahren nach einem der Ansprüche 1 - 12, ferner umfassend:
Empfangen einer Benutzereingabe, die einen Bereich mindestens eines der ersten und zweiten Kieferabschnitte angibt, und Zuweisen einer maximalen Frästiefe zu diesem Bereich; und
Erzeugen mindestens eines modifizierten 3D-Modells, das den mindestens einen der ersten und zweiten Kieferabschnitte darstellt, auf der Grundlage des angegebenen Bereichs und der maximalen Frästiefe,
wobei das automatische Anpassen mindestens eines der anderen Freiheitsgrade das Begrenzen der Abstände zwischen gegenüberliegenden Oberflächen des ersten und des zweiten Kieferabschnitts auf der Grundlage des mindestens einen modifizierten 3D-Modells umfasst, so dass diese positiv sind.

14. Verfahren nach Anspruch 13, wobei die GUI ein Okklusogramm von mindestens einem des ersten Kieferabschnitts und des zweiten Kieferabschnitts auf der Grundlage des mindestens einen modifizierten 3D-Modells umfasst.

15. Computergestütztes Verfahren zur Bildung einer chirurgischen Führung auf der Grundlage einer bestimmten virtuellen Okklusion, umfassend:
Bestimmen einer virtuellen Okklusion zwischen einem ersten Kieferabschnitt eines Patienten und einem zweiten Kieferabschnitts des Patienten unter Verwendung des Verfahrens nach einem der Ansprüche 1 - 14; und
Bilden einer chirurgischen Führung, um die Zähne des Patienten während einer orthognathen Operation in die bestimmte Okklusion zu führen.

16. Verfahren nach Anspruch 15, wobei das Bilden der chirurgischen Führung das Herstellen der chirurgischen Führung mittels einer additiven Fertigungstechnologie umfasst.

17. Verfahren nach Anspruch 15 oder 16, wobei die chirurgische Führung einen Führungskörper umfasst, wobei der Führungskörper eine Oberseite, eine der Oberseite gegenüberliegende Unterseite und eine Kante umfasst, wobei die Oberseite und die Unterseite Vertiefungen umfassen, die komplementär zu Oberflächenabschnitten des ersten und des zweiten Kieferabschnitts des Patienten in der bestimmten Okklusion geformt sind.

18. System zum Bestimmen einer virtuellen Okklusion, umfassend:
eine grafische Benutzeroberfläche (GUI); und
einen Prozessor, der so konfiguriert ist, dass er das Verfahren nach einem der Ansprüche 1 - 17 ausführt.

19. Nicht-transitorisches, computerlesbares Speichermedium mit darin gespeicherten Anweisungen, die, wenn sie von einem Computergerät ausgeführt werden, das Computergerät veranlassen, das Verfahren gemäß einem der Ansprüche 1 - 17 auszuführen.

## Revendications

1. Procédé mis en œuvre par ordinateur de détermination d'une occlusion virtuelle, comprenant :
l'obtention (200) d'une représentation en 3D d'une première partie de mâchoire d'un patient et d'une seconde partie de mâchoire d'un patient, la seconde partie de mâchoire étant opposée à et mobile par rapport à la première partie de mâchoire ;
à l'aide de la représentation en 3D, la définition et la représentation (202) dans une interface graphique utilisateur (GUI pour « Graphic User Interface ») d'une position initiale de la seconde partie de mâchoire par rapport à la première partie de mâchoire, la position initiale étant définie par un point de contrôle sur la seconde partie de mâchoire et la seconde partie de mâchoire présentant six degrés de liberté prédéterminés par rapport à un système de coordonnées dont l'origine est fixe par rapport à la première partie de mâchoire ;
la réception (204) d'une entrée utilisateur de modifications d'au moins l'un des degrés de liberté, l'entrée utilisateur de modifications comprenant une modification d'une position du point de contrôle par rapport à la seconde partie de mâchoire ;
le réglage automatique (206) d'au moins l'un des autres degrés de liberté afin de minimiser la distance verticale entre le point de contrôle et l'origine tout en contraignant les distances entre les surfaces opposées des première et seconde parties de mâchoire à être positives, déterminant ainsi une occlusion entre les première et seconde parties de mâchoire ; et
la représentation (208), dans la GUI, des première et seconde parties de mâchoire dans l'occlusion déterminée.

2. Procédé selon la revendication 1, dans lequel l'entrée utilisateur de modifications comprend en outre une modification d'au moins l'une parmi : une position de la seconde partie de mâchoire dans une direction du système de coordonnées correspondant à la direction gauche-droite du patient, la position de la seconde partie de mâchoire dans une direction du système de coordonnées correspondant à la direction antéro-postérieure du patient, ou une rotation de la seconde partie de mâchoire autour d'une direction du système de coordonnées correspondant à la direction ascendante-descendante du patient ; et
dans lequel le réglage automatique d'au moins l'un des autres degrés de liberté comprend le réglage automatique d'au moins l'une parmi : une position de la seconde partie de mâchoire dans une direction du système de coordonnées correspondant à la direction ascendante-descendante du patient, une rotation de la seconde partie de mâchoire autour d'une direction du système de coordonnées correspondant à la direction gauche-droite du patient, ou une rotation de la seconde partie de mâchoire autour d'une direction du système de coordonnées correspondant à la direction antéro-postérieure du patient.

3. Procédé selon la revendication 1, dans lequel l'entrée utilisateur de modifications comprend en outre une modification d'au moins l'une parmi : une position de la seconde partie de mâchoire dans une direction du système de coordonnées correspondant à la direction gauche-droite du patient, la position de la seconde partie de mâchoire dans une direction du système de coordonnées correspondant à la direction antéro-postérieure du patient, une rotation de la seconde partie de mâchoire autour d'une direction du système de coordonnées correspondant à la direction ascendante-descendante du patient, ou une rotation de la seconde partie de mâchoire autour d'une direction du système de coordonnées correspondant à la direction gauche-bas du patient ; et
dans lequel le réglage automatique d'au moins l'un des autres degrés de liberté comprend le réglage automatique d'au moins l'une parmi : une position de la seconde partie de mâchoire dans une direction du système de coordonnées correspondant à la direction ascendante-descendante du patient, ou une rotation de la seconde partie de mâchoire autour d'une direction du système de coordonnées correspondant à la direction antéro-postérieure du patient.

4. Procédé selon l'une quelconque des revendications 1 à 3, dans lequel l'entrée utilisateur de modifications pour modifier la position du point de contrôle par rapport à la seconde partie de mâchoire comprend la modification de la position du point de contrôle dans la direction du système de coordonnées correspondant à la direction antéro-postérieure du patient.

5. Procédé selon la revendication 1, dans lequel l'entrée utilisateur de modifications comprend en outre une modification d'au moins l'une parmi : une position de la seconde partie de mâchoire dans une direction du système de coordonnées correspondant à la direction mésiale-distale du patient, la position de la seconde partie de mâchoire dans une direction du système de coordonnées correspondant à la direction bucco-linguale du patient, une rotation de la seconde partie de mâchoire autour d'une direction du système de coordonnées correspondant à la direction ascendante-descendante du patient, ou une rotation de la seconde partie de mâchoire autour d'une direction du système de coordonnées correspondant à la direction mésiale-distale du patient ; et
dans lequel le réglage automatique d'au moins l'un des autres degrés de liberté comprend le réglage automatique d'au moins l'une parmi : une position de la seconde partie de mâchoire dans une direction du système de coordonnées correspondant à la direction ascendante-descendante du patient, ou une rotation de la seconde partie de mâchoire autour d'une direction du système de coordonnées correspondant à la direction bucco-linguale du patient.

6. Procédé selon l'une quelconque des revendications 1 à 5, dans lequel :
la première partie de mâchoire comprend au moins une partie du maxillaire du patient, et
la seconde partie de mâchoire comprend au moins une partie de la mandibule du patient.

7. Procédé selon l'une quelconque des revendications 1 à 5, dans lequel :
la première partie de mâchoire comprend au moins une partie de la mandibule du patient, et
la seconde partie de mâchoire comprend au moins une partie du maxillaire du patient.

8. Procédé selon l'une quelconque des revendications 1 à 7, dans lequel la GUI comprend un modèle 3D de chacune de la première partie de mâchoire et de la seconde partie de mâchoire.

9. Procédé selon la revendication 8, comprenant en outre l'affichage d'un ou plusieurs parmi :
une distance entre les première et seconde parties de mâchoire dans l'occlusion déterminée, sous forme d'une palette de couleurs sur le modèle 3D ;
les points de contact entre les première et seconde parties de mâchoire dans l'occlusion déterminée sur le modèle 3D ;
les points de contact approximatifs entre les première et seconde parties de mâchoire dans l'occlusion déterminée sur le modèle 3D ; et
une coque convexe autour des points de contact et/ou des points de contact approximatifs entre les première et seconde parties de mâchoire dans l'occlusion déterminée sur le modèle 3D.

10. Procédé selon l'une quelconque des revendications 1 à 9, dans lequel la GUI comprend en outre un occlusogramme d'au moins l'une parmi la première partie de mâchoire et la seconde partie de mâchoire.

11. Procédé selon la revendication 10, comprenant en outre l'affichage d'un ou plusieurs parmi :
les points de contact entre les première et seconde parties de mâchoire dans l'occlusion déterminée sur l'occlusogramme ;
les points de contact approximatifs entre les première et seconde parties de mâchoire dans l'occlusion déterminée sur l'occlusogramme ; et
une coque convexe autour des points de contact et/ou des points de contact approximatifs entre les première et seconde parties de mâchoire dans l'occlusion déterminée sur l'occlusogramme.

12. Procédé selon l'une quelconque des revendications 1 à 11, comprenant en outre la mise à jour de la représentation des première et seconde parties de mâchoire dans la GUI dans l'occlusion déterminée en réponse à une entrée utilisateur supplémentaire de modifications d'au moins l'un des degrés de liberté et aux réglages automatiques correspondants d'au moins l'un des autres degrés de liberté.

13. Procédé selon l'une quelconque des revendications 1 à 12, comprenant en outre :
la réception d'une entrée utilisateur indiquant une zone d'au moins l'une parmi les première et seconde parties de mâchoire et l'attribution d'une profondeur maximale de fraisage à ladite zone ; et
la génération d'au moins un modèle 3D modifié représentant l'au moins une parmi les première et seconde parties de mâchoire sur la base de la zone indiquée et de la profondeur maximale de fraisage,
dans lequel le réglage automatique d'au moins l'un des autres degrés de liberté comprend la contrainte des distances entre les surfaces opposées des première et seconde parties de mâchoire pour qu'elles soient positives sur la base de l'au moins un modèle 3D modifié.

14. Procédé selon la revendication 13, dans lequel la GUI comprend un occlusogramme d'au moins l'une parmi la première partie de mâchoire et la seconde partie de mâchoire basé sur l'au moins un modèle 3D modifié.

15. Procédé mis en œuvre par ordinateur de création d'un guide chirurgical sur la base d'une occlusion virtuelle déterminée, comprenant :
la détermination d'une occlusion virtuelle entre une première partie de mâchoire d'un patient et la seconde partie de mâchoire du patient à l'aide du procédé selon l'une quelconque des revendications 1 à 14 ; et
la création d'un guide chirurgical destiné à guider les dents du patient vers l'occlusion déterminée au cours d'une chirurgie orthognathique.

16. Procédé selon la revendication 15, dans lequel la création du guide chirurgical comprend la fabrication du guide chirurgical au moyen d'une technologie de fabrication additive.

17. Procédé selon la revendication 15 ou 16, dans lequel le guide chirurgical comprend un corps de guide, ledit corps de guide comprenant une surface supérieure, une surface inférieure opposée à la surface supérieure et un bord, lesdites surfaces supérieure et inférieure comprenant des indentations de forme complémentaire aux parties de surface des première et seconde parties de mâchoire du patient dans l'occlusion déterminée.

18. Système de détermination d'une occlusion virtuelle, comprenant :
une interface utilisateur graphique (GUI) ; et
un processeur configuré pour exécuter le procédé selon l'une quelconque des revendications 1 à 17.

19. Support de stockage non transitoire lisible par ordinateur, sur lequel sont stockées des instructions qui, lorsqu'elles sont exécutées par un dispositif informatique, amènent le dispositif informatique à mettre en œuvre le procédé selon l'une quelconque des revendications 1 à 17.
